(19) Europäisches Patentamt
European Patent Office
Office européen des brevets

(11) **EP 4 775 530 A1**

(12) **EUROPEAN PATENT APPLICATION**
published in accordance with Art. 153(4) EPC

(43) Date of publication:
**15.07.2026 Bulletin 2026/29**

(21) Application number: 24862896.8

(22) Date of filing: **06.09.2024**

(51) International Patent Classification (IPC):
**B82B 3/00** (2006.01)    **B01F 33/3011** (2022.01)
**B01J 13/02** (2006.01)    **B01J 19/00** (2006.01)
**C12N 15/88** (2006.01)

(52) Cooperative Patent Classification (CPC):
**B01F 33/3011; B01J 13/02; B01J 19/00;
B82B 3/00; C12N 15/88**

(86) International application number:
**PCT/JP2024/031996**

(87) International publication number:
**WO 2025/053247 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **08.09.2023 JP 2023146373**

(71) Applicants:
• **Toyama Prefecture
Toyama-shi,
Toyama 930-8501 (JP)**
• **Toyama Prefectural University
Imizu-shi, Toyama, 939-0398 (JP)**

• **Zeon Corporation
Tokyo 100-8246 (JP)**

(72) Inventors:
• **TAKATA, Koji
Nanto-shi, Toyama 939-1503 (JP)**
• **MURAKAMI, Tatsuya
Imizu-shi, Toyama 939-0398 (JP)**
• **HASHIOKA, Shingi
Tokyo 100-8246 (JP)**

(74) Representative: **Maiwald GmbH
Elisenhof
Elisenstraße 3
80335 München (DE)**

(54) **NANOPARTICLE PRODUCTION METHOD**

(57)    There is provided a method for manufacturing nanoparticles in which desired nanoparticles can be readily manufactured using a microfluidic device.

A method for manufacturing nanoparticles according to the present invention includes introducing a first liquid containing at least one material into a mixing channel 20 through a first fluid inlet channel 10a of a microfluidic device 1a, and introducing a second liquid into the mixing channel 20 through a second fluid inlet channel 10b and a third fluid inlet channel 10c on both sides of the first fluid inlet channel 10a. A Reynolds number in the mixing channel 20 is set to a predetermined value or greater, and a flow of the first liquid in the mixing channel 20 becomes asymmetric such that the flow of the first liquid immediately after entering the mixing channel 20 through the first fluid inlet channel 10a is biased toward one of two side walls of the mixing channel 20.

FIG.4A

**Description**

TECHNICAL FIELD

The present invention relates to a method for manufacturing nanoparticles using a microfluidic device.

BACKGROUND ART

As a method for manufacturing nanoparticles that can be used as carriers in drug delivery systems (DDS) or as pharmaceuticals, a manufacturing method using a microfluidic device has been known. For example, Patent Document 1 describes a technique for chemosynthetically manufacturing reconstituted HDL (rHDL) having approximately the same size (around 10 nm) as that of natural high-density lipoprotein (HDL) by mixing a first liquid containing a phospholipid (DMPC: dimyristoylphosphatidylcholine) with a second liquid containing apolipoprotein A-I (apoA-I) using a microfluidic device.

In the method for manufacturing nanoparticles described in Patent Document 1, a microfluidic device including three fluid inlet channels (a central fluid inlet channel and fluid inlet channels on both sides thereof) and a mixing channel where the fluid inlet channels merge is used to manufacture rHDL. In the microfluidic device, the first liquid is introduced into the central fluid inlet channel, and the second liquid is introduced into the fluid inlet channels on both sides thereof.

The first liquid flows into the mixing channel through the central fluid inlet channel, and the second liquid flows into the mixing channel through the fluid inlet channels on both sides thereof. It is considered that the first liquid and the second liquid form a microvortex, which is symmetric (mirror-symmetric) with respect to the central fluid inlet channel, in the mixing channel, thereby rapidly mixing the DMPC contained in the first liquid with the apolipoprotein A-I contained in the second liquid and reconstituting rHDL.

In recent years, it has been clear that rHDL using distearoylphosphatidylcholine (DSPC) as a phospholipid (DSPC-rHDL) is promising as a carrier in a drug delivery system or as a pharmaceutical. Conventionally, a cholic acid dialysis method has been known as a technique for reconstituting DSPC-rHDL. However, the cholic acid dialysis method has problems in that the time and effort are required for the work of removing cholic acid by dialysis, and further, toxicity arises due to residual cholic acid.

Therefore, the present inventors have attempted to reconstitute DSPC-rHDL using the method for manufacturing nanoparticles described in Patent Document 1. However, DSPC-rHDL cannot be reconstituted by the method for manufacturing nanoparticles described in Patent Document 1, and it has been suggested that the cause of this failure is insufficient mixing efficiency of the first liquid and the second liquid in the mixing channel.

In addition, it is considered that phospholipids having high phase transition temperatures, such as distearoylphosphatidylcholine (DSPC) and hydrogenated soy phosphatidylcholine (HSPC), undergo rapid self-assembly during the mixing of the first liquid and the second liquid, and therefore, it is considered that reconstitution of rHDL using the method for manufacturing nanoparticles described in Patent Document 1 cannot be achieved or is difficult. Therefore, there has been a demand for a technique capable of manufacturing various nanoparticles (rHDL, liposomes, lipid nanoparticles, polymer nanoparticles, polymer-lipid hybrids, and the like), which can be used as carriers in drug delivery systems, pharmaceuticals, cosmetics, or quasi-drugs, using the microfluidic device.

CITATION LIST

PATENT DOCUMENT

Patent Document 1: United States Patent No. 10864162

DISCLOSURE OF THE INVENTION

PROBLEM TO BE SOLVED BY THE INVENTION

The present invention has been made in view of such circumstances, and an object of the present invention is to provide a method for manufacturing nanoparticles in which desired nanoparticles can be readily manufactured using a microfluidic device.

MEANS FOR SOLVING PROBLEM

In order to achieve the above-described object, a method for manufacturing nanoparticles according to the present invention includes introducing a first liquid containing at least one material into a mixing channel through a first fluid inlet channel of a microfluidic device, and introducing a second liquid into the mixing channel through a second fluid inlet channel and a third fluid inlet channel on both sides of the first fluid inlet channel. A Reynolds number in the mixing channel is set to a predetermined value or greater, and a flow of the first liquid in the mixing channel becomes asymmetric such that the flow of the first liquid immediately after entering the mixing channel through the first fluid inlet channel is biased toward one of two side walls of the mixing channel.

In the method for manufacturing nanoparticles according to the present invention, the Reynolds number in the mixing channel is set to the predetermined value or greater, and the flow of the first liquid in the mixing channel becomes asymmetric such that the flow of the first liquid immediately after entering the mixing channel through the first fluid inlet channel is biased toward one of the two side walls of the mixing channel. Therefore, it is considered that the first liquid and the second liquid are more easily mixed at the initial stage where the first liquid and the second liquid flow into the mixing channel; therefore, the mixing efficiency of the first liquid and the second liquid is improved.

Accordingly, rapid and uniform mixing of the first liquid and the second liquid is promoted, so that fine nanoparticles can be readily manufactured based on the at least one material contained in the first liquid. Particularly, the method for manufacturing nanoparticles according to the present embodiment is suitable, for example, for manufacturing fine nanoparticles having a particle size of around 10 nm, and it is confirmed that, in the method for manufacturing nanoparticles according to the present invention, the reconstitution of rHDL using DSPC as a phospholipid, which is difficult to manufacture using conventional methods for manufacturing nanoparticles using microfluidic devices, is enabled.

The Reynolds number may be 151 or greater. In this case, the greater the Reynolds number becomes, the more easily the flow of the first liquid is biased toward one of the two side walls of the mixing channel at the initial stage where the first liquid flows into the mixing channel (on the upstream side of the mixing channel). Therefore, the mixing efficiency of the first liquid and the second liquid is improved, so that fine nanoparticles can be readily manufactured.

The Reynolds number may be 281 or greater. In this case, due to the formation of a microvortex, the boundary between the region where the first liquid and the second liquid are mixed and the regions on both sides thereof (the regions mainly containing the second liquid) more easily disappears. Therefore, the first liquid and the second liquid are more easily mixed between these regions, so that the mixing efficiency of the first liquid and the second liquid is further improved. Accordingly, rapid and uniform mixing of the first liquid and the second liquid is promoted, so that fine nanoparticles can be readily manufactured.

A spacing between the first fluid inlet channel and the second fluid inlet channel may be greater than a lateral width of each of the first fluid inlet channel, the second fluid inlet channel, and the third fluid inlet channel, and a spacing between the first fluid inlet channel and the third fluid inlet channel may be greater than the lateral width of each of the first fluid inlet channel, the second fluid inlet channel, and the third fluid inlet channel. In this case, due to the formation of a microvortex, the boundary between the region where the first liquid and the second liquid are mixed and the regions on both sides thereof (the regions mainly containing the second liquid) more easily disappears. Accordingly, rapid and uniform mixing of the first liquid and the second liquid is promoted, so that fine nanoparticles can be readily manufactured.

The method for manufacturing nanoparticles according to the present invention may further include discharging the first liquid and the second liquid from the mixing channel through a fluid outlet channel located downstream of the mixing channel. In addition, the fluid outlet channel may be composed of a single fluid outlet channel having a lateral width narrower than a lateral width of the mixing channel.

In this case, since the first liquid and the second liquid are concentrated in the single main fluid outlet channel, the Reynolds number in the fluid outlet channel increases, and the mixing of the first liquid and the second liquid in the fluid outlet channel is promoted. Accordingly, in addition to the mixing channel, the first liquid and the second liquid are also mixed in the fluid outlet channel, so that the mixing efficiency of the first liquid and the second liquid is further improved.

A temperature of the mixing channel may be 45°C or higher. In this case, in the mixing channel, aggregates of the at least one material contained in the first liquid are less likely to occur, so that fine nanoparticles can be more easily formed compared to conventional methods for manufacturing nanoparticles.

It should be noted that a temperature of the mixing channel may be room temperature or higher (for example, 10°C or higher, preferably 15°C or higher, or 20°C or higher). In this case as well, it is established by experiments conducted by the present inventors that, in the mixing channel, aggregates of the at least one material contained in the first liquid is less likely to occur; therefore, fine nanoparticles are more easily formed compared to conventional methods for manufacturing nanoparticles.

The at least one material may contain a lipid. In the method for manufacturing nanoparticles according to the present invention, for example, phospholipids (DMPC, DPPC, DSPC, DAPC, HSPC, and the like) can be used as the at least one material contained in the first liquid.

The second liquid may be an aqueous solvent, and may contain at least one water-soluble material. In the method for manufacturing nanoparticles according to the present invention, for example, apolipoprotein A-I (apoA-I) can be used as the at least one water-soluble material contained in the second liquid.

The at least one water-soluble material may contain at least one of a protein and a peptide. For example, by mixing the first liquid containing phospholipid (DSPC) with the second liquid containing apolipoprotein A-I (apoA-I), DSPC-rHDL having a fine particle size that can be used as a carrier in a drug delivery system or as a pharmaceutical can be reconstituted.

BRIEF DESCRIPTION OF DRAWINGS

Fig. 1 is an exploded perspective view of a microfluidic device or the like used in a method for manufacturing nanoparticles according to one embodiment of the present invention;

Fig. 2 is a plan view of the microfluidic device shown in Fig. 1;

Fig. 3 is a diagram showing a state in which the microfluidic device or the like shown in Fig. 1 is immersed in a constant-temperature water bath;

Fig. 4(a) is a conceptual diagram showing the state of flow of a first liquid and a second liquid flowing through a mixing channel, in the microfluidic device shown in Fig. 2, when the Reynolds number in the mixing channel is a first predetermined value or greater;

Fig. 4(b) is a conceptual diagram showing the state of flow of the first liquid and the second liquid flowing through the mixing channel, in the microfluidic device shown in Fig. 2, when the Reynolds number in the mixing channel is a second predetermined value or greater;

Fig. 4(c) is a conceptual diagram showing the state of flow of the first liquid and the second liquid flowing through a mixing channel, in the microfluidic device shown in Fig. 2, when the Reynolds number in the mixing channel is less than the first predetermined value;

Fig. 5(a) is a photograph showing the state of the first liquid and the second liquid flowing through the mixing channel when the Reynolds number in the mixing channel is approximately 151;

Fig. 5(b) is a photograph showing the state of the first liquid and the second liquid flowing through the mixing channel when the Reynolds number in the mixing channel is approximately 173;

Fig. 5(c) is a photograph showing the state of the first liquid and the second liquid flowing through the mixing channel when the Reynolds number in the mixing channel is approximately 281;

Fig. 5(d) is a photograph showing the state of the first liquid and the second liquid flowing through the mixing channel when the Reynolds number in the mixing channel is approximately 306;

Fig. 5(e) is a photograph showing the state of the first liquid and the second liquid flowing through the mixing channel when the Reynolds number in the mixing channel is approximately 345;

Fig. 5(f) is a photograph showing the state of the first liquid and the second liquid flowing through the mixing channel when the Reynolds number in the mixing channel is approximately 383;

Fig. 5(g) is a photograph showing the state of the first liquid and the second liquid flowing through the mixing channel when the Reynolds number in the mixing channel is approximately 479;

Fig. 5(h) is a photograph showing the state of the first liquid and the second liquid flowing through the mixing channel when the Reynolds number in the mixing channel is approximately 575;

Fig. 5(i) is a photograph showing the state of the first liquid and the second liquid flowing through the mixing channel when the Reynolds number in the mixing channel is approximately 108;

Fig. 5(j) is a photograph showing the state of the first liquid and the second liquid flowing through the mixing channel when the Reynolds number in the mixing channel is approximately 130;

Fig. 6(a) is a photograph showing the state of the first liquid and the second liquid when the flow rate ratio of the second liquid introduced into the second fluid inlet channel to the second fluid introduced into the third fluid inlet channel is 10:7, and the Reynolds number in the mixing channel is approximately 175;

Fig. 6(b) is a photograph showing the state of the first liquid and the second liquid flowing through the mixing channel when the Reynolds number in the mixing channel is approximately 197;

Fig. 6(c) is a photograph showing the state of the first liquid and the second liquid flowing through the mixing channel when the Reynolds number in the mixing channel is approximately 316;

Fig. 6(d) is a photograph showing the state of the first liquid and the second liquid flowing through the mixing channel when the Reynolds number in the mixing channel is approximately 395;

Fig. 6(e) is a photograph showing the state of the first liquid and the second liquid flowing through the mixing channel when the Reynolds number in the mixing channel is approximately 474;

Fig. 7 is a photograph showing the state of the first liquid and the second liquid flowing through the mixing channel when the total flow rate of the first liquid and the second liquid is 5.5 mL/min, and the temperature of the mixing channel is approximately 55°C;

Fig. 8 is a graph showing the results of size exclusion chromatography when the total flow rate of the first liquid and the second liquid is set to each of 5.5 mL/min, 7.7 mL/min, 8.8 mL/min, 11 mL/min, and 13.2 mL/min;

Fig. 9 is a graph showing the results of size exclusion chromatography when the temperature of the mixing channel is set to approximately 45°C and approximately 55°C;

Fig. 10 is a photograph showing the state of the first liquid and the second liquid flowing through the mixing channel when a single main fluid outlet channel is restricted such that a fluid is discharged from the mixing channel through the fluid outlet channel;

Fig. 11 is a graph showing the results of size exclusion chromatography when the single fluid outlet channel is restricted such that the fluid is discharged from the mixing channel through the main fluid outlet channel;

Fig. 12 is an exploded perspective view of a microfluidic device or the like used in a method for manufacturing nanoparticles according to another embodiment of the present invention;

Fig. 13 is a plan view of the microfluidic device shown in Fig. 12;

Fig. 14(a) is a photograph showing the state of the first liquid and the second liquid flowing through the mixing channel when the Reynolds number in the mixing channel is approximately 1437, using the microfluidic device shown in Fig. 13;

Fig. 14(b) is a photograph showing the state of the first liquid and the second liquid flowing through the mixing channel when the Reynolds number in the mixing channel is approximately 2395, using the microfluidic device shown in Fig. 13;

Fig. 14(c) is a photograph showing the state of the first liquid and the second liquid flowing through the mixing channel when the Reynolds number in the mixing channel is approximately 1081, using the microfluidic device shown in Fig. 13;

Fig. 15 is a plan view of another pattern of the microfluidic device shown in Fig. 12;

Fig. 16 is a photograph showing the state of the first liquid and the second liquid flowing through the mixing channel when the Reynolds number in the mixing channel is approximately 1081, using the microfluidic device shown in Fig. 15;

Fig. 17 is a plan view of another pattern of the microfluidic device shown in Fig. 12;

Fig. 18 is a photograph showing the state of the first liquid and the second liquid flowing through the mixing channel when the Reynolds number in the mixing channel is approximately 1081, using the microfluidic device shown in Fig. 17; and

Fig. 19 is a graph showing the results of size exclusion chromatography when the temperature of the mixing channel is set to approximately 20°C and the Reynolds number is set to 1081, using the device shown in Figs. 12 and 13.

BEST MODES FOR CARRYING OUT THE INVENTION

Hereinafter, embodiments of the present invention will be described with reference to the drawings. It should be noted that the illustrated content is a merely schematic and exemplary representation for understanding the present invention, and the appearance, dimensional ratios, or the like may differ from the actual product. In addition, the present invention is not limited to the following embodiments.

First embodiment

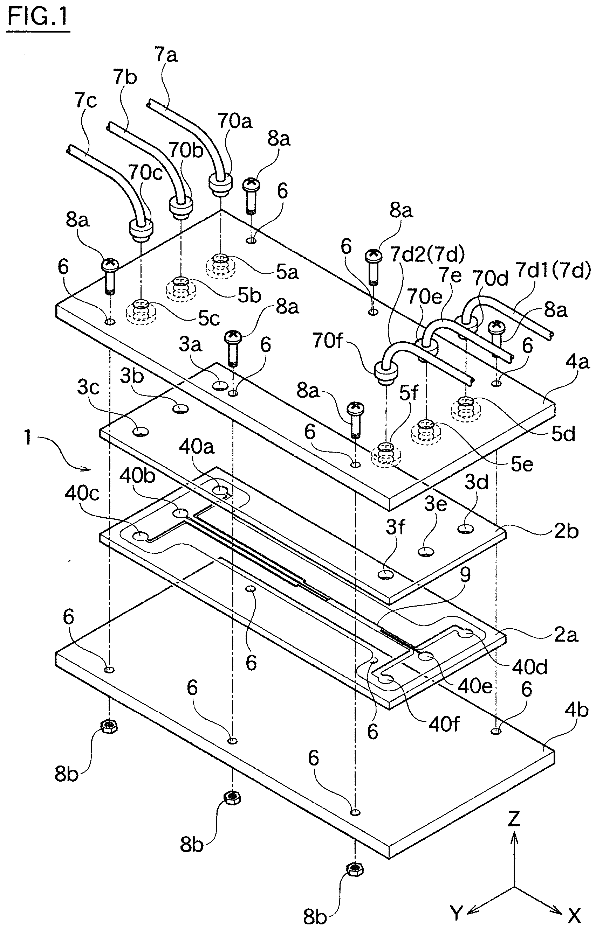

A microfluidic device 1 shown in Fig. 1 is a device used in a method for manufacturing nanoparticles according to one embodiment of the present invention, and is used to manufacture nanoparticles that can be used, for example, as a carrier in a drug delivery system, a pharmaceutical, a cosmetic, or a quasi-drug.

The microfluidic device 1 includes a base chip 2a in which a groove 9 is formed, and a cover chip 2b that covers the base chip 2a. A clamping plate 4a and a clamping plate 4b are fixed to the microfluidic device 1 by a plurality of screws 8a and a plurality of nuts 8b. Tubes 7a to 7e are fixed to the clamping plate 4a through connectors 70a to 70f. Hereinafter, details of these members will be described.

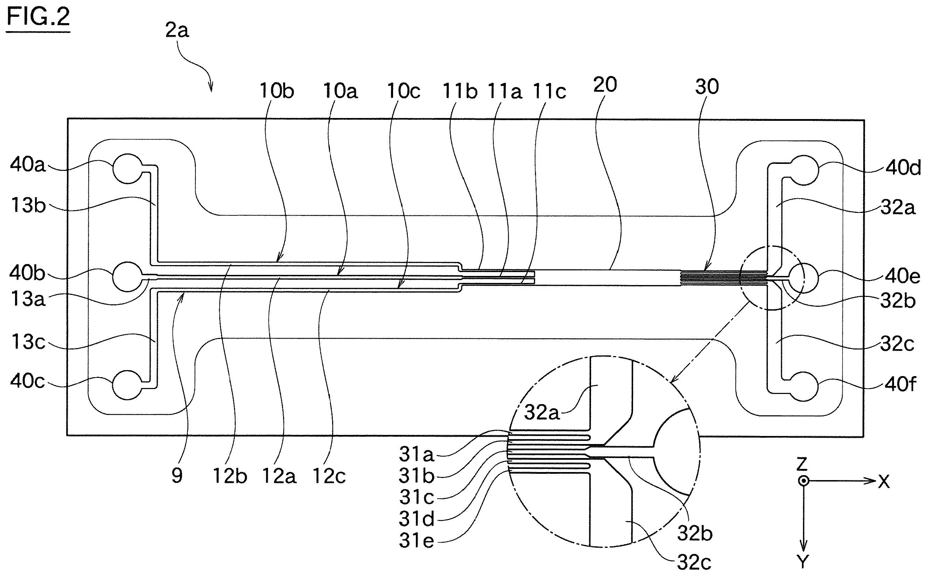

It should be noted that, in Figs 1 and 2, an X-axis is an axis parallel to the longitudinal direction of the base chip 2a, a Y-axis is an axis parallel to the transverse direction of the base chip 2a, and a Z-axis is an axis perpendicular to the X-axis and the Y-axis. The X-axis, the Y-axis, and the Z-axis are orthogonal to each other. Hereinafter, the positive Z-axis side is referred to as an upper side, and the negative Z-axis side is referred to as a lower side.

The base chip 2a is formed as a plate having a flat shape, and includes the groove 9. The groove 9 is formed on one main surface (the upper surface) of the base chip 2a. The base chip 2a is made of any material suitable for the flow of a fluid passing through the groove 9. The material constituting the base chip 2a, although not particularly limited, is polymer, plastic (polypropylene, polydimethylsiloxane, polycarbonate, polyethylene terephthalate, acrylic resin, or the like), glass, silicon, ceramic, metal, or the like.

The cover chip 2b is formed as a plate similar to the base chip 2a, and is superimposed on the base chip 2a so as to cover the surface of the base chip 2a on which the groove 9 is formed. A closed space along the groove 9 is formed between the base chip 2a and the cover chip 2b by stacking the cover chip 2b on the base chip 2a. Accordingly, the fluid can flow through the interior of the groove 9 without leaking from the groove 9.

The cover chip 2b is made of the same material as the base chip 2a, but may be made of a material different from that of the base chip 2a. Through-holes 3a to 3c are formed at a constant spacing along the Y-axis at one end portion of the cover chip 2b in an X-axis direction. Through-holes 3d to 3f are formed at a constant spacing along the Y-axis at the other end portion of the cover chip 2b in the X-axis direction.

The clamping plates 4a and 4b are each formed as a plate having a flat shape, and clamp the base chip 2a and the cover chip 2b from above and below. Accordingly, the base chip 2a and the cover chip 2b are fixed (pressed) from above and below by the clamping plates 4a and 4b. The material constituting the clamping plates 4a and 4b, although not particularly limited, is polymer, plastic (polypropylene, polydimethylsiloxane, polycarbonate, polyethylene terephthalate, acrylic resin, or the like), glass, silicon, ceramic, metal, or the like.

Through-holes 5a to 5c are formed at a constant spacing along the Y-axis at one end portion of the clamping plate 4a in the X-axis direction. Through-holes 5d to 5f are formed at a constant spacing along the Y-axis at the other end portion of the clamping plate 4a in the X-axis direction.

The connectors 70a to 70c attached to one ends of the tubes 7a to 7c are fitted into the through-holes 5a to 5c of the clamping plate 4a. At least a part of the connectors 70a to 70c penetrates through the through-holes 5a to 5c, and is fitted into the through-holes 3a to 3c of the cover chip 2b. The connectors 70d to 70f attached to one ends of the tubes 7d (7d1 and 7d2) and 7e are fitted into the through-holes 5d to 5f of the clamping plate 4a. At least a part of the connectors 70d to 70f penetrates through the through-holes 5d to 5f, and is fitted into the through-holes 3d to 3f of the cover chip 2b.

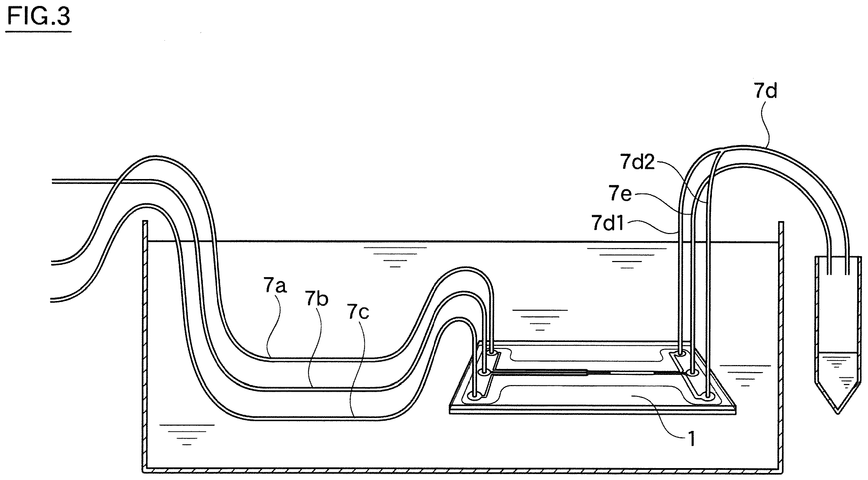

The tubes 7a to 7e, although not particularly limited, are made of plastic such as polytetrafluoroethylene (PTFE). The tube 7d1 and the tube 7d2 are formed as one tube 7d (Fig. 3) that branches into two conduits, but may be formed as separate tubes. The connectors 70a to 70f, although not particularly limited, are made of plastic or the like.

Three screw holes 6 are formed at a constant spacing along the X-axis at one end portion of the clamping plate 4a in a Y-axis direction. Three screw holes 6 are formed at a constant spacing along the X-axis at the other end portion of the clamping plate 4a in the Y-axis direction. In addition, three screw holes 6 are formed at a constant spacing along the X-axis at one end portion of the clamping plate 4b in the Y-axis direction. Three screw holes 6 are formed at a constant spacing along the X-axis at the other end portion of the clamping plate 4b in the Y-axis direction.

The clamping plates 4a and 4b are fixed by six screws 8a and six nuts 8b in a state in which the clamping plates 4a and 4b clamp the base chip 2a and the cover chip 2b from above and below. The screws 8a penetrate through the screw holes 6 formed in the clamping plate 4a, and penetrate through the screw holes 6 formed in the clamping plate 4b. The nuts 8b screw to the screws 8a on the lower surface side of the clamping plate 4b. Means for fixing the clamping plates 4a and 4b is not limited to the screws 8a and the nuts 8b, and may be other fasteners such as rivets or studs.

The base chip 2a and the cover chip 2b are clamped from above and below by the clamping plates 4a and 4b, and further the clamping plates 4a and 4b were fixed by the screws 8a and the nuts 8b, thereby improving the airtightness of the closed space (the groove 9) formed between the base chip 2a and the cover chip 2b.

As shown in Fig. 2, the groove 9 includes at least a first fluid inlet channel 10a, a second fluid inlet channel 10b, a third fluid inlet channel 10c, and a mixing channel 20. The groove 9 further includes a fluid outlet channel 30 and terminal portions 40a to 40f in addition to these channels; however, the configuration of the groove 9 is not limited thereto. The depth of the groove 9 is not particularly limited, but is 0.05 to 0.5 mm or 0.1 to 0.3 mm. In the present embodiment, the mixing channel 20 is a channel with a rectangular cross-section. The Reynolds number which will be described later is calculated assuming a flow in a channel with a rectangular cross-section.

The terminal portions 40a to 40c are formed at a constant spacing along the Y-axis at one end portion of the base chip 2a in the X-axis direction. The terminal portions 40d to 40f are formed at a constant spacing along the Y-axis at the other end portion of the base chip 2a in the X-axis direction. The terminal portions 40a to 40f have a circular shape in plan view. However, the shape of the terminal portions 40a to 40f in plan view may be an elliptical shape, a quadrilateral shape, another polygonal shape, another shape, or the like.

As shown in Fig. 1, the terminal portions 40a to 40f are located directly below the through-holes 3a to 3f. The through-holes 5a to 5f formed in the clamping plate 4a, the through-holes 3a to 3f formed in the cover chip 2b, and the terminal portions 40a to 40f formed in the base chip 2a communicate with each other. Therefore, the fluid flowing through the tubes 7a to 7c flows into the first fluid inlet channel 10a to the third fluid inlet channel 10c (Fig. 2) through the terminal portions 40a to 40c.

In addition, the fluid flowing through the fluid outlet channel 30 (Fig. 2) flows out into the tubes 7d (7d1 and 7d2) and 7e through the terminal portions 40d to 40f. The length of the tubes 7d to 7e is not particularly limited, but is 0.2 to 2.0 mm. The inner diameter of the tubes 7d to 7e is not particularly limited, but is 0.1 to 1.5 mm.

As shown in Fig. 2, the first fluid inlet channel 10a includes at least a main fluid inlet channel 11a. The first fluid inlet channel 10a includes a downstream expansion channel 12a and an upstream expansion channel 13a in addition to the main fluid inlet channel 11a; however, the configuration of the first fluid inlet channel 10a is not limited thereto.

In addition, the second fluid inlet channel 10b includes at least a main fluid inlet channel 11b. The second fluid inlet channel 10b includes a downstream expansion channel 12b and an upstream expansion channel 13b in addition to the main fluid inlet channel 11b; however, the configuration of the second fluid inlet channel 10b is not limited thereto.

In addition, the third fluid inlet channel 10c includes at least a main fluid inlet channel 11c. The third fluid inlet channel 10c includes a downstream expansion channel 12c and an upstream expansion channel 13c in addition to the main fluid inlet channel 11c; however, the configuration of the third fluid inlet channel 10c is not limited thereto.

The first fluid inlet channel 10a is formed at the center of the base chip 2a in the Y-axis direction, and is located between the second fluid inlet channel 10b and the third fluid inlet channel 10c. The second fluid inlet channel 10b and the third fluid inlet channel 10c are located on both sides (outside) of the first fluid inlet channel 10a in the Y-axis direction. The second fluid inlet channel 10b and the third fluid inlet channel 10c have a shape that is symmetric (mirror-symmetric) with respect to the first fluid inlet channel 10a.

Each of the main fluid inlet channel 11a, the main fluid inlet channel 11b, and the main fluid inlet channel 11c extends linearly along the X-axis. The respective lateral widths (the channel widths) of the main fluid inlet channel 11a, the main fluid inlet channel 11b, and the main fluid inlet channel 11c are equal, but may be different. The lateral width of each of the main fluid inlet channel 11a, the main fluid inlet channel 11b, and the main fluid inlet channel 11c is not particularly limited, but is 0.02 to 0. 3 mm.

The respective lengths (the channel lengths) of the main fluid inlet channel 11a, the main fluid inlet channel 11b, and the main fluid inlet channel 11c are equal, but may be different. The length of each of the main fluid inlet channel 11a, the main fluid inlet channel 11b, and the main fluid inlet channel 11c is not particularly limited, but is 2.0 to 8.0 mm.

A spacing D1 (the center-to-center distance) between the main fluid inlet channel 11a and the main fluid inlet channel 11b is greater than a maximum lateral width W1 among the respective lateral widths of the main fluid inlet channel 11a, the main fluid inlet channel 11b, and the main fluid inlet channel 11c. The ratio D1/W1 of D1 to W1 is not particularly limited, but may satisfy, for example, $1 < D1/W1 \leq 7$. It should be noted that the spacing D1 represents a center-to-center distance between the channels. A spacing D2 which will be described later also represents a center-to-center distance between the channels.

Similarly, the spacing D2 (the center-to-center distance) between the main fluid inlet channel 11a and the main fluid inlet channel 11c is greater than the maximum lateral width W1 among the respective lateral widths of the main fluid inlet channel 11a, the main fluid inlet channel 11b, and the main fluid inlet channel 11c. The ratio D2/W1 of D2 to W1 is not particularly limited, but may satisfy, for example, $1 < D2/W1 \leq 7$.

The downstream expansion channel 12a is connected to the upstream end portion of the main fluid inlet channel 11a. The downstream expansion channel 12a extends linearly along the X-axis. The length of the downstream expansion channel 12a is longer than the length of the main fluid inlet channel 11a, and the lateral width of the downstream expansion channel 12a is greater than the lateral width of the main fluid inlet channel 11a.

The downstream expansion channel 12b is connected to the upstream end portion of the main fluid inlet channel 11b. The majority of the downstream expansion channel 12b extends linearly along the X-axis. However, the downstream end portion of the downstream expansion channel 12b is bent at a right angle. The length of the downstream expansion channel 12b (the length from one end to the other end of the downstream expansion channel 12b) is longer than the length of the main fluid inlet channel 11b, and the lateral width of the downstream expansion channel 12b is greater than the lateral width of the main fluid inlet channel 11b.

The downstream expansion channel 12c is connected to the upstream end portion of the main fluid inlet channel 11c. The majority of the downstream expansion channel 12c extends linearly along the X-axis. However, the downstream end portion of the downstream expansion channel 12c is bent at a right angle. The length of the downstream expansion channel 12c (the length from one end to the other end of the downstream expansion channel 12c) is longer than the length of the main fluid inlet channel 11c, and the lateral width of the downstream expansion channel 12c is greater than the lateral width of the main fluid inlet channel 11c.

The upstream expansion channel 13a is connected to the upstream end portion of the downstream expansion channel 12a. The upstream expansion channel 13a extends linearly along the X-axis. The length of the upstream expansion channel 13a is shorter than the length of the downstream expansion channel 12a, and the lateral width of the upstream expansion channel 13a is greater than the lateral width of the downstream expansion channel 12a. The upstream end portion of the upstream expansion channel 13a is connected to the terminal portion 40b.

The upstream expansion channel 13b is connected to the upstream end portion of the downstream expansion channel 12b. The majority of the upstream expansion channel 13b extends linearly along the Y-axis. However, the upstream end portion of the upstream expansion channel 13b is bent at a right angle. The length of the upstream expansion channel 13b (the length from one end to the other end of the upstream expansion channel 13b) is shorter than the length of the downstream expansion channel 12b (the length from the one end to the other end of the downstream expansion channel 12b). The lateral width of the upstream expansion channel 13b is greater than the lateral width of the downstream expansion channel 12b. The upstream end portion of the upstream expansion channel 13b is connected to the terminal portion 40a.

The upstream expansion channel 13c is connected to the upstream end portion of the downstream expansion channel 12c. The majority of the upstream expansion channel 13c extends linearly along the Y-axis. However, the upstream end portion of the upstream expansion channel 13c is bent at a right angle. The length of the upstream expansion channel 13c (the length from one end to the other end of the upstream expansion channel 13c) is shorter than the length of the downstream expansion channel 12c (the length from the one end to the other end of the downstream expansion channel 12c). The lateral width of the upstream expansion channel 13c is greater than the lateral width of the downstream expansion channel 12c. The upstream end portion of the upstream expansion channel 13c is connected to the terminal portion 40c.

The addition of the downstream expansion channel 12a and the upstream expansion channel 13a to the main fluid inlet channel 11a causes the lateral width of the first fluid inlet channel 10a to become narrower in a stepwise manner from the upstream side toward the downstream side. Accordingly, the first fluid inlet channel 10a can be prevented from being clogged, and the fluid can be introduced into the mixing channel 20 through the first fluid inlet channel 10a at a desired flow velocity.

Similarly, the addition of the downstream expansion channel 12b and the upstream expansion channel 13b to the main fluid inlet channel 11b causes the lateral width of the second fluid inlet channel 10b to become narrower in a stepwise manner from the upstream side toward the downstream side. Accordingly, the second fluid inlet channel 10b can be prevented from being clogged, and the fluid can be introduced into the mixing channel 20 through the second fluid inlet channel 10b at a desired flow velocity.

Similarly, the addition of the downstream expansion channel 12c and the upstream expansion channel 13c to the main fluid inlet channel 11c causes the lateral width of the third fluid inlet channel 10c to become narrower in a stepwise manner from the upstream side toward the downstream side. Accordingly, the third fluid inlet channel 10c can be prevented from being clogged, and the fluid can be introduced into the mixing channel 20 through the third fluid inlet channel 10c at a desired flow velocity.

The mixing channel 20 is connected to the downstream end portion of each of the first fluid inlet channel 10a to the third fluid inlet channel 10c. The fluid outlet channel 30 is connected to the downstream end portion of the mixing channel 20. The lateral width of the mixing channel 20 is greater than the lateral width of each of the main fluid inlet channel 11a, the main fluid inlet channel 11b, and the main fluid inlet channel 11c. The lateral width of the mixing channel 20 is not particularly limited, but is 0.2 to 3 mm or 0.5 to 1.5 mm.

In addition, the length of the mixing channel 20 is not particularly limited, but is 5 to 20 mm or 5 to 15 mm. The depth of the mixing channel 20 is not particularly limited, but is 0.05 to 0.5 mm or 0.1 to 0.3 mm. The fluids flowing out from the main fluid inlet channel 11a, the main fluid inlet channel 11b, and the main fluid inlet channel 11c merge and are mixed in the mixing channel 20.

The ratio w/h of the lateral width w (the width along the Y-axis) of the mixing channel to the depth h of the mixing channel 20 along the Z-axis is particularly limited, but is preferably 1 or more, 2 or more, or 3 to 7. However, this numerical range applies to the case where the main fluid inlet channels 11a to 11c are arranged along the Y-axis, and when the main fluid inlet channels 11a to 11c are arranged along the Z-axis on the upstream side of the mixing channel 20, it is preferable that the ratio of h/w is within this range. In addition, in the present embodiment, it is preferable that the Z-axis substantially coincides with the direction of gravity; however, the X-axis, the Y-axis, or another intermediate axis may substantially coincide with the direction of gravity.

It should be noted that the above dimensional relationship is a dimensional relationship in one embodiment, and may differ from the above numerical range in the case of industrial production or the like. However, it is important that the configuration is such that the flow of a first liquid entering the mixing channel 20 from the main fluid inlet channel 11a is made asymmetric within the mixing channel 20.

The fluid that has flowed into the mixing channel 20 is discharged from the mixing channel 20 through the fluid outlet channel 30. The fluid outlet channel 30 includes a plurality of main fluid outlet channels (in the present embodiment, main fluid outlet channels 31a to 31e). However, the number of the main fluid outlet channels is not limited to five, and may be one, two to four, or six or more. The fluid outlet channel 30 further includes a plurality of outlet expansion channels (in the present embodiment, outlet expansion channels 32a to 32c) in addition to these channels; however, the configuration of the fluid outlet channel 30 is not limited thereto. The number of the outlet expansion channels 32a to 32c is not limited to three, and may be one, two, or four or more.

The main fluid outlet channels 31a to 31e all extend linearly along the X-axis, and are disposed at a constant spacing along the Y-axis. The main fluid outlet channels 31a to 31e are connected to the downstream end portion of the mixing channel 20.

The lateral width of each of the main fluid outlet channels 31a to 31e is smaller than the lateral width of the mixing channel 20. The lateral width of each of the main fluid outlet channels 31a to 31e is equal to the lateral width of each of the main fluid inlet channels 11a to 11c, but may be smaller or greater than that.

The lateral width of each of the main fluid outlet channels 31a to 31e is not particularly limited, but is 0.05 to 0.3 mm. The length of each of the main fluid outlet channels 31a to 31e is shorter than the length of the mixing channel 20, but is not particularly limited. The ratio W2/W3 of the lateral width W2 of each of the main fluid outlet channels 31a to 31e to the lateral width W3 of the mixing channel 20 is not particularly limited, but satisfies $1/10 \leq W2/W3 \leq 1/5$.

The outlet expansion channel 32a is connected to the main fluid outlet channels 31a and 31b on the upstream side of the outlet expansion channel 32a, and is connected to the terminal portion 40d on the downstream side of the outlet expansion channel 32a. The fluids that have flowed out from the main fluid outlet channels 31a and 31b merge in the outlet expansion channel 32a, and flow to the terminal portion 40d. The lateral width of the outlet expansion channel 32a is greater than the lateral width of each of the main fluid outlet channels 31a and 31b.

The outlet expansion channel 32b is connected to the main fluid outlet channel 31c on the upstream side of the outlet expansion channel 32b, and is connected to the terminal portion 40e on the downstream side of the outlet expansion channel 32b. The fluid that has flowed out from the main fluid outlet channel 31c flows to the terminal portion 40e through the outlet expansion channel 32b. The lateral width of the outlet expansion channel 32b is greater than the lateral width of the main fluid outlet channel 31c.

The outlet expansion channel 32c is connected to the main fluid outlet channels 31d and 31e on the upstream side of the outlet expansion channel 32c, and is connected to the terminal portion 40f on the downstream side of the outlet expansion channel 32c. The fluids that have flowed out from the main fluid outlet channels 31d and 31e merge in the outlet expansion channel 32c, and flow to the terminal portion 40f. The lateral width of the outlet expansion channel 32c is greater than the lateral width of each of the main fluid outlet channels 31d and 31e.

As shown in Fig. 3, the microfluidic device 1 is immersed in a constant-temperature water bath in a state in which the tubes 7a to 7e are attached. Accordingly, the microfluidic device 1, the tubes 7a to 7e, and the fluid flowing through the interiors thereof are heated to a predetermined temperature. The temperature of the fluid stored in the constant-temperature water bath (the temperature of the microfluidic device 1 (in more detail, the first fluid inlet channel 10a to the third fluid inlet channel 10c, the mixing channel 20, and the fluid outlet channel 30), the tubes 7a to 7e, and the fluid flowing through the interiors thereof is room temperature or higher (for example, 10°C or higher, preferably 15°C or higher, or 20°C or higher), preferably 45°C or higher, or more preferably 55°C or higher.

As shown in Fig. 2, a method for manufacturing nanoparticles according to the present embodiment includes a step of introducing the first liquid containing at least one material into the mixing channel 20 through the first fluid inlet channel 10a of the microfluidic device 1, and introducing a second liquid into the mixing channel 20 through the second fluid inlet channel 10b and the third fluid inlet channel 10c on both sides of the first fluid inlet channel 10a.

The first liquid is a solution containing at least one material. Examples of the material contained in the first liquid include lipids, polyethylene glycol-modified lipids, and biodegradable synthetic polymers, and the like. Examples of lipids include cholesterol, fatty acids, phospholipids, glycerolipids, sphingolipids, sterol lipids, prenol lipids, saccharolipids, and the like, and phospholipids are preferred.

Examples of phospholipids include phosphatidylglycerol, phosphatidylserine, phosphatidylcholine, phosphatidylethanolamine, and the like, and phosphatidylcholine is preferred. Examples of phosphatidylcholine include dimyristoylphosphatidylcholine (DMPC), dipalmitoylphosphatidylcholine (DPPC), distearoylphosphatidylcholine

(DSPC), diarachidoylphosphatidylcholine (DAPC), hydrogenated soy phosphatidylcholine (HSPC), egg phosphatidylcholine (EPC), 1-palmitoyl-2-oleoylphosphatidylcholine (POPC), and dioleoylphosphatidylcholine (DOPC). These lipids are dissolved in a suitable solvent such as methanol, ethanol, or chloroform. In addition, examples of biodegradable synthetic polymers include polylactic-co-glycolic acid (PLGA), polylactic acid, polyglycolic acid, polydioxanone, and the like.

The second liquid is an aqueous solvent. The second liquid may contain at least one water-soluble material. Examples of water-soluble materials include proteins, peptides, and polyethylene glycol-modified lipids, and the like. Preferably, the second liquid contains at least one of a protein and a peptide.

Examples of proteins include apolipoproteins and their genetically modified apolipoproteins. Examples of apolipoproteins include apolipoproteins belonging to the group of apolipoproteins A to E, for example, at least one selected from the group consisting of apolipoprotein A-I (apoA-I), apolipoprotein A-II (apoA-II), apolipoprotein C (apoC), apolipoprotein E (apoE), and their genetically modified apolipoproteins. Of these, apolipoprotein A-I (apoA-I) and its genetically modified apoA-I are preferred.

A genetically modified apolipoprotein means a variant, analog, or the like, (for example, a functional equivalent) having a function equivalent to the function of an apolipoprotein (for example, a lipid-binding function). Examples of genetically modified apolipoproteins include partial fragments of apolipoproteins and apolipoproteins that are a combination thereof. N-terminal 43 amino acid-deleted apoA-I is provided as an example of a genetically modified apolipoprotein A-I.

Examples of peptides include apoA-I partial peptides, apoA-I mimetic peptides, and the like."

As shown in Figs. 2 and 3, the first liquid is introduced into the microfluidic device 1 through the tube 7b, for example, by a syringe pump. The second liquid is introduced into the microfluidic device 1 through the tubes 7a and 7c, for example, by a syringe pump. The first liquid flows into the first fluid inlet channel 10a through the terminal portion 40b. The second liquid flows into the second fluid inlet channel 10b and the third fluid inlet channel 10c through the terminal portions 40a and 40c.

In addition, the first liquid is introduced into the mixing channel 20 through the first fluid inlet channel 10a (the upstream expansion channel 13a, the downstream expansion channel 12a, and the main fluid inlet channel 11a). In addition, the second liquid is introduced into the mixing channel 20 through the second fluid inlet channel 10b (the upstream expansion channel 13b, the downstream expansion channel 12b, and the main fluid inlet channel 11b) and the third fluid inlet channel 10c (the upstream expansion channel 13c, the downstream expansion channel 12c, and the main fluid inlet channel 11c).

Further, the first liquid and the second liquid are mixed in the mixing channel 20, and are discharged from the mixing channel 20 through the fluid outlet channel 30. In more detail, a portion of the fluid flowing through the mixing channel 20 is discharged from the mixing channel 20 through the main fluid outlet channels 31a and 31b, and further flows into the tube 7d1 through the outlet expansion channel 32a and the terminal portion 40d.

In addition, a portion of the fluid flowing through the mixing channel 20 is discharged from the mixing channel 20 through the main fluid outlet channel 31c, and further flows into the tube 7e through the outlet expansion channel 32b and the terminal portion 40e. In addition, a portion of the fluid flowing through the mixing channel 20 is discharged from the mixing channel 20 through the main fluid outlet channels 31d and 31e, and further flows into the tube 7d2 through the outlet expansion channel 32c and the terminal portion 40f.

Next, as shown in Fig. 3, the fluids (the first liquid and the second liquid) that have flowed into the tubes 7d and 7e are stored in a container such as a test tube.

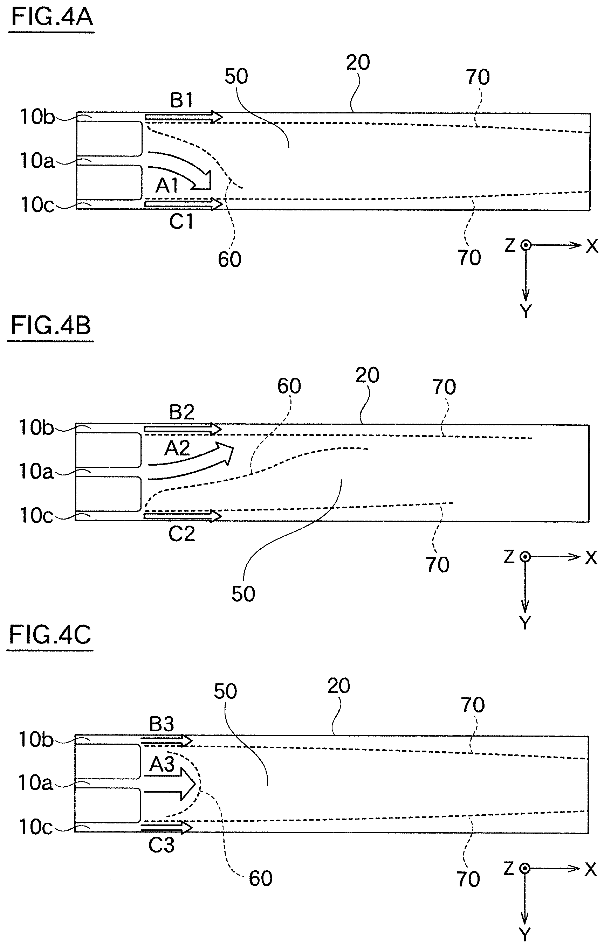

In the present embodiment, the Reynolds number in the mixing channel 20 shown in Fig. 2 is set to a predetermined value or greater. When the Reynolds number in the mixing channel 20 is set to a first predetermined value, it is considered that the flow of the fluid as will be described below occurs in the mixing channel 20. As shown in Fig. 4(a), the first liquid immediately after entering the mixing channel 20 through the first fluid inlet channel 10a flows on average in the direction indicated by arrow A1, that is, toward one of the two side walls of the mixing channel 20.

In addition, the second liquid immediately after entering the mixing channel 20 through the second fluid inlet channel 10b flows along the direction indicated by arrow B1, that is, along the extending direction of the mixing channel 20. In addition, the second liquid immediately after entering the mixing channel 20 through the third fluid inlet channel 10c flows along the direction indicated by arrow C1, that is, along the extending direction of the mixing channel 20.

As a result, the flow of the first liquid immediately after entering the mixing channel 20 through the first fluid inlet channel 10a becomes asymmetric with respect to the first fluid inlet channel 10a such that the flow of the first liquid is biased toward one of the two side walls of the mixing channel 20. In the example shown in Fig. 4(a), the flow of the first liquid is biased toward the side wall on the positive Y-axis side of the mixing channel 20, but may also be biased toward the side wall on the negative Y-axis side of the mixing channel 20.

In a microvortex region 50, a microvortex asymmetric with respect to the first fluid inlet channel 10a is generated, and the first liquid and the second liquid are mixed. Here, a first boundary 60 asymmetric with respect to the first fluid inlet channel 10a is formed between a flow mainly containing the first liquid (a region around arrow A1) and the microvortex region 50. In addition, a second boundary 70 is formed between the microvortex region 50 and a flow mainly containing the second liquid on both sides of the microvortex region 50 in the Y-axis direction.

It is considered that the flow of the first liquid immediately after entering the mixing channel 20 through the first fluid inlet channel 10a becomes asymmetric, and the average flow directions of the first liquid and the second liquid are no longer parallel to each other; therefore, the first liquid and the second liquid are more easily mixed even at the initial stage where the first liquid and the second liquid flow into the mixing channel 20.

That is, the first liquid and the second liquid are mixed not only in the microvortex region 50 but also on the upstream side of the mixing channel 20 (in the vicinity of the first fluid inlet channel 10a, the second fluid inlet channel 10b, and the third fluid inlet channel 10c). This mixing may be observable as an ultra-microvortex (vortex smaller than the above-described microvortex).

It should be noted that the first predetermined value described above is, as one example, 151, but is not particularly limited as long as the flow of the first liquid in the mixing channel 20 can be made asymmetric in the manner shown in Fig. 4(a). In addition, bubbles may be mixed into the mixing channel 20. Even if bubbles are mixed into the mixing channel 20, as long as the flow of the fluid is confirmed as generally shown in Fig. 4(a), the same operational effect (the effect that the first liquid and the second liquid are more easily mixed at the initial stage where the first liquid and the second liquid flow into the mixing channel 20) as when no bubbles are present in the mixing channel 20 is obtained.

That is, even when bubbles are mixed into the mixing channel 20, as long as the flow of the fluid is confirmed as generally shown in Fig. 4(a), the flow of the first liquid is asymmetric with respect to the first fluid inlet channel 10a such that the flow of the first liquid is biased toward one of the two side walls of the mixing channel 20. In addition, the Reynolds number Re in the mixing channel 20 is calculated based on the following Equation (1). Here, $\rho$ is the fluid density. In addition, U is the average fluid velocity. In addition, $\mu$ is the fluid viscosity. In addition, w is the channel width of the mixing channel 20. In addition, h is the channel depth of the mixing channel 20. In addition, Q is the flow rate.

[Equation 1]

$$Re = \frac{\rho}{\mu} \frac{2Q}{w+h} = \frac{\rho U}{\mu} \frac{2wh}{w+h} \quad (1)$$

In addition, when the Reynolds number in the mixing channel 20 is set to a second predetermined value (a value higher than the first predetermined value, it is considered that the flow of the fluid as will be described below occurs in the mixing channel 20. As shown in Fig. 4(b), the first liquid immediately after entering the mixing channel 20 through the first fluid inlet channel 10a flows on average in the direction indicated by arrow A2, that is, toward one of the two side walls of the mixing channel 20.

In addition, the second liquid immediately after entering the mixing channel 20 through the second fluid inlet channel 10b flows along the direction indicated by arrow B2, that is, along the extending direction of the mixing channel 20. In addition, the second liquid immediately after entering the mixing channel 20 through the third fluid inlet channel 10c flows along the direction indicated by arrow C2, that is, along the extending direction of the mixing channel 20.

As a result, the flow of the first liquid immediately after entering the mixing channel 20 through the first fluid inlet channel 10a becomes asymmetric with respect to the first fluid inlet channel 10a such that the flow of the first liquid is biased toward one of the two side walls of the mixing channel 20. The first liquid and the second liquid are mixed in the microvortex region 50; however, a microvortex may not be observable.

Here, the first boundary 60 asymmetric with respect to the first fluid inlet channel 10a is formed between a flow mainly containing the first liquid (a region around arrow A2) and the microvortex region 50. In addition, the second boundary 70 is formed between the microvortex region 50 and a flow mainly containing the second liquid on both sides of the microvortex region 50 in the Y-axis direction.

It is considered that the flow of the first liquid immediately after entering the mixing channel 20 through the first fluid inlet channel 10a becomes asymmetric, and the average flow directions of the first liquid and the second liquid are no longer parallel to each other; therefore, the first liquid and the second liquid are more easily mixed even at the initial stage where the first liquid and the second liquid flow into the mixing channel 20. That is, the first liquid and the second liquid are mixed not only in the microvortex region 50 but also on the upstream side of the mixing channel 20 (in the vicinity of the first fluid inlet channel 10a, the second fluid inlet channel 10b, and the third fluid inlet channel 10c). This mixing may be observable as an ultra-microvortex.

Additionally, the second boundary 70 disappears (becomes indistinct or is disturbed) midway through the mixing channel 20. This indicates that uniform mixing is promoted between the inner side and the outer side of the second boundary 70 in the Y-axis direction, and the mixing efficiency of the first liquid and the second liquid is further improved. This disappearance of the second boundary 70 occurs further upstream in the mixing channel 20 as the Reynolds number increases above the second predetermined value as the same temperature. In addition, when the Reynolds number increases, the microvortex becomes indistinct, and an ultra-microvortex different from conventional microvortices is observed. This indicates that the mixing of the first liquid and the second liquid proceeds more rapidly.

It should be noted that the second predetermined value described above is, as one example, 281, but is not particularly limited as long as the flow of the first liquid in the mixing channel 20 can be made asymmetric in the manner shown in Fig. 4(b). In addition, bubbles may be mixed into the mixing channel 20.

Even if bubbles are mixed into the mixing channel 20, as long as the flow of the fluid is confirmed as generally shown in Fig. 4(b), the same operational effects ((i) the effect that the first liquid and the second liquid are more easily mixed at the initial stage where the first liquid and the second liquid flow into the mixing channel 20, and (ii) the effect that the mixing efficiency of the first liquid and the second liquid is improved as the second boundary 70 disappears midway through the mixing channel 20) as when no bubbles are present in the mixing channel 20 are obtained. That is, even when bubbles are mixed into the mixing channel 20, as long as the flow of the fluid is confirmed as generally shown in Fig. 4(b), the flow of the first liquid is asymmetric with respect to the first fluid inlet channel 10a such that the flow of the first liquid is biased toward one of the two side walls of the mixing channel 20.

Particularly, the method for manufacturing nanoparticles according to the present embodiment is suitable, for example, for manufacturing fine nanoparticles having a particle size of 8 to 15 nm, and the reconstruction of rHDL using DSPC as a phospholipid, which is difficult to manufacture using conventional methods for manufacturing nanoparticles using microfluidic devices, is enabled (details will be described in Examples).

In addition, the method for manufacturing nanoparticles according to the present embodiment is suitable for reconstituting rHDL using, for example, a phospholipid other than DSPC, such as hydrogenated soy phosphatidylcholine (HSPC), where the phospholipid has a higher phase transition temperature than DMPC.

Therefore, various nanoparticles (rHDL, liposomes, lipid nanoparticles, polymer nanoparticles, polymer-lipid hybrids, and the like), which can be used as carriers in drug delivery systems, pharmaceuticals, cosmetics, or quasi-drugs can be manufactured, using the microfluidic device.

If the Reynolds number in the mixing channel 20 is set to be less than the first predetermined value, it is considered that the flow of the fluid as will be described below occurs in the mixing channel 20. As shown in Fig. 4(c), the first liquid immediately after entering the mixing channel 20 through the first fluid inlet channel 10a flows on average along the direction indicated by arrow A3, that is, along the extending direction of the mixing channel 20.

In addition, the second liquid immediately after entering the mixing channel 20 through the second fluid inlet channel 10b flows along the direction indicated by arrow B3, that is, along the extending direction of the mixing channel 20. In addition, the second liquid immediately after entering the mixing channel 20 through the third fluid inlet channel 10c flows along the direction indicated by arrow C3, that is, along the extending direction of the mixing channel 20.

As a result, the flow of the first liquid immediately after entering the mixing channel 20 through the first fluid inlet channel 10a becomes symmetric with respect to the first fluid inlet channel 10a. In addition, in the microvortex region 50, a microvortex symmetric with respect to the first fluid inlet channel 10a is generated, and the first liquid and the second liquid are mixed.

Here, the first boundary 60 symmetric with respect to the first fluid inlet channel 10a is formed between a flow mainly containing the first liquid (a region around arrow A3) and the microvortex region 50. In addition, the second boundary 70 is formed between the microvortex region 50 and a flow mainly containing the second liquid on both sides of the microvortex region 50 in the Y-axis direction.

In this case, since the average flow directions of the first liquid and the second liquid become parallel to each other at the initial stage where the first liquid and the second liquid flow into the mixing channel 20, the first liquid and the second liquid are difficult to mix. That is, the first and second liquids are mixed mainly in the microvortex region 50 by a microvortex, and are difficult to mix on the upstream side of the mixing channel 20 (in the vicinity of the first fluid inlet channel 10a, the second fluid inlet channel 10b, and the third fluid inlet channel 10c). Therefore, the mixing efficiency of the first liquid and the second liquid may not be sufficient, and it may be difficult to realize rapid and uniform mixing of the first liquid and the second liquid.

In the present embodiment, as shown in Fig. 2, the spacing between the main fluid inlet channel 11a and the main fluid inlet channel 11b is greater than the lateral width of each of the main fluid inlet channel 11a, the main fluid inlet channel 11b, and the main fluid inlet channel 11c. In addition, the spacing between the main fluid inlet channel 11a and the main fluid inlet channel 11c is greater than the lateral width of each of the main fluid inlet channel 11a, the main fluid inlet channel 11b, and the main fluid inlet channel 11c.

Therefore, the boundary (the second boundary 70) between the microvortex region 50 and a flow mainly containing the second liquid on both sides of the microvortex region 50 in the Y-axis direction more easily disappears as far upstream as possible. Accordingly, rapid and uniform mixing of the first liquid and the second liquid is promoted, so that fine nanoparticles can be readily manufactured.

It should be noted that the present invention is not limited to the above-described embodiment, and can be modified in various ways within the scope of the present invention. For example, the following modifications can be implemented.

As shown in Fig. 2, the fluid outlet channel 30 includes five main fluid outlet channels 31a to 31e, but may include only a single main fluid outlet channel having a lateral width narrower than the lateral width of the mixing channel 20. For example, the fluid outlet channel 30 may include only the main fluid outlet channel 31c. In this case, the fluid is discharged from the mixing channel 20 through the single main fluid outlet channel.

In addition, the main fluid outlet channels 31a, 31b, 31d, and 31e may be blocked by a jig or the like such that the fluid does not flow. Alternatively, the outlet expansion channels 32a and 32c may be blocked by a jig or the like such that the fluid does not flow through the main fluid outlet channels 31a, 31b, 31d, and 31e. Alternatively, the tube 7d (the tubes 7d1 and 7d2) shown in Fig. 1 may be blocked by a jig or the like such that the fluid does not flow through the main fluid outlet channels 31a, 31b, 31d, and 31e.

In this case, in effect, the fluid outlet channel 30 includes only a single main fluid outlet channel (the main fluid outlet channel 31c). It should be noted that the blocking positions of the main fluid outlet channels 31a, 31b, 31d, and 31e may be at the upstream end portions of the main fluid outlet channels 31a, 31b, 31d, and 31e, or may be at the downstream end portions.

Alternatively, the blocking positions of the main fluid outlet channels 31a, 31b, 31d, and 31e may be any positions between the upstream end portions and the downstream end portions of the main fluid outlet channels 31a, 31b, 31d, and 31e. The ratio W2/W3 of the lateral width W2 of the single main fluid outlet channel 31c to the lateral width W3 of the mixing channel 20 satisfies $1/20 \le W2/W3 \le 1$ or $1/10 \le W2/W3 \le 1/2$.

In this way, when the fluid outlet channel 30 includes the single main fluid outlet channel 31c, the first liquid and the second liquid are concentrated in the single main fluid outlet channel 31c; therefore, the Reynolds number in the main fluid outlet channel 31c increases, and the mixing of the first liquid and the second liquid in the main fluid outlet channel 31c is promoted. Accordingly, in addition to the mixing channel 20, the first liquid and the second liquid are also mixed in the main fluid outlet channel 31c, so that the mixing efficiency of the first liquid and the second liquid is further enhanced.

The number of the main fluid outlet channels is not limited to five, and may be four or less or six or more. When the number of the main fluid outlet channels is four or less, for example, the fluid outlet channel 30 may include any two main fluid outlet channels, any three main fluid outlet channels, or any four main fluid outlet channels among the main fluid outlet channels 31a to 31e. In this case, of the main fluid outlet channels 31a to 31e, the main fluid outlet channel 31c may be made essential.

In addition, as shown in Fig. 2, the first fluid inlet channel 10a includes the downstream expansion channel 12a and the upstream expansion channel 13a; however, these channels may be omitted. In this case, the first fluid inlet channel 10a may be composed of only the main fluid inlet channel 11a. In addition, the length of the main fluid inlet channel 11a may be extended as needed. The same applies to the second fluid inlet channel 10b and the third fluid inlet channel 10c.

In addition, as shown in Fig. 2, the fluid outlet channel 30 includes the outlet expansion channels 32a to 32c; however, these channels may be omitted. In addition, the groove 9 need only include at least the first fluid inlet channel 10a to the third fluid inlet channel 10c (the main fluid inlet channels 11a to 11c) and the mixing channel 20, and may not include the fluid outlet channel 30 and the terminal portions 40a to 40f.

When the fluid outlet channel 30 and/or the terminal portions 40a to 40f are omitted from the groove 9, the first liquid and the second liquid may be discharged directly or indirectly (through the tubes and the like) from the downstream side of the mixing channel 20. In this case as well, the effects described in the embodiment can be obtained.

Second embodiment

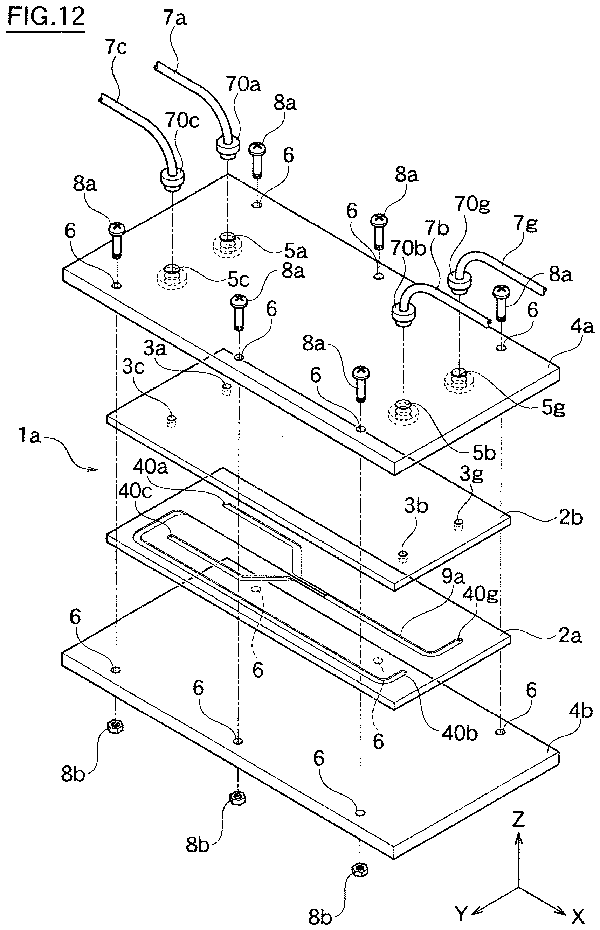

A microfluidic device 1a shown in Fig. 12 is a device used in a method for manufacturing nanoparticles according to another embodiment of the present invention. The description of portions common to the above-described embodiment is partially omitted, and in the following description, portions that differ from the above-described embodiment will be mainly described. It should be noted that, in the drawings of the above-described embodiment and the following embodiments, common members are denoted by common reference signs.

In addition, at least a part of the configuration of the following embodiments (the second and subsequent embodiments) can be replaced or combined with at least a part of the configuration of the above-described embodiment, and at least a part of the configuration of the above-described embodiment can be replaced or combined with at least a part of the configuration of the following embodiments.

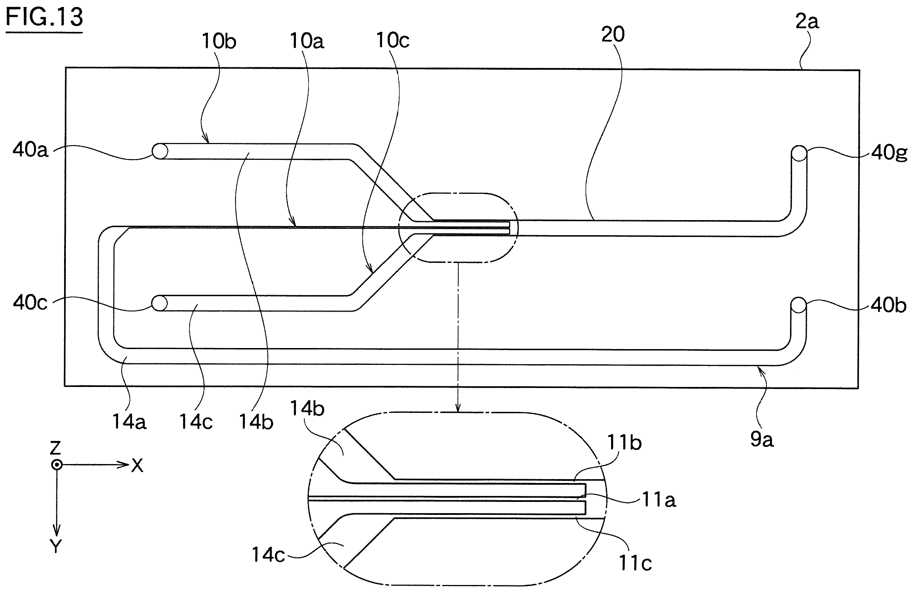

As shown in Figs. 12 and 13, the microfluidic device 1a of the present embodiment differs from the microfluidic device of the above-described embodiment mainly in that the configuration of a groove 9a differs from the configuration of the groove 9 shown in Figs. 1 and 2.

As shown in Fig. 13, the groove 9a includes at least the first fluid inlet channel 10a, the second fluid inlet channel 10b, the third fluid inlet channel 10c, and the mixing channel 20. The first fluid inlet channel 10a includes an expansion channel 14a having a channel cross-section larger than that of the main channel 11a, in addition to the main fluid inlet channel 11a. Similarly, the second fluid inlet channel 10b includes an expansion channel 14b having a channel cross-section larger than that of the main channel 11b, in addition to the main fluid inlet channel 11b. Similarly, the third fluid inlet channel 10c includes an expansion channel 14c having a channel cross-section larger than that of the main channel 11c, in addition to the main fluid inlet channel 11c.

It should be noted that, as in the first embodiment, the channel is not formed by the groove 9a alone; instead, the opening of the groove 9a is closed by the lower surface of the cover chip along the Z-axis, thereby forming a channel that is sealed except for the inlet and the outlet. In the present embodiment, as shown in Fig. 13, one end on the downstream side of each of the main fluid inlet channels 11a, 11b, and 11c communicates with the mixing channel 20, and the other ends on the upstream side of the main fluid inlet channels 11a, 11b, and 11c communicate with the expansion channels 14a, 14b, and 14c, respectively.

The respective channel cross-sectional areas of the expansion channels 14a, 14b, and 14c may differ from each other, but are preferably approximately the same, and for example, may be approximately the same as the channel cross-sectional area of the mixing channel 20. The channel cross-sectional area of the mixing channel 20 can be set to, for example, 0.1 to 1 mm$^2$. In addition, the respective channel cross-sectional areas of the main fluid inlet channels 11a, 11b, and 11c may differ from each other, but are preferably substantially equal, and it is preferable that each channel cross-sectional area is within the range of 1/20 to 1/5 of the channel cross-sectional area of the mixing channel 20.

The terminal portions 40a to 40c for introducing the fluid are formed at the upstream ends of the expansion channels 14a to 14c. In addition, a terminal portion 40g for extracting the fluid is formed at the downstream end of the mixing channel 20. The terminal portions 40a to 40c and 40g are formed at a part of the groove 9a, and communicate with the through-holes 3a to 3c and 3g, respectively, which are formed in the cover chip 2b shown in Fig. 12.

The through-holes 3a to 3c and 3g communicate with the through-holes 5a to 5c and 5g, respectively, which are formed in the clamping plate 4a. The tubes 7a to 7c and 7g are attached to the through-holes 5a to 5c and 5g through the adapters 70a to 70c and 70g, respectively, and communicate therewith, respectively.

As shown in Fig. 13, in the present embodiment, the terminal portions 40a and 40c are formed at the one end portion of the base chip 2a in the X-axis direction, and are disposed at a constant spacing along the Y-axis. The terminal portions 40b and 40g are formed at the other end portion of the base chip 2a in the X-axis direction, and are disposed at a constant spacing along the Y-axis.

As shown in Fig. 12, the terminal portions 40a to 40c and 40g are located directly below the through-holes 3a to 3c and 3g. The through-holes 5a to 5c and 5g formed in the clamping plate 4a, the through-holes 3a to 3c and 3g formed in the cover chip 2b, and the terminal portions 40a to 40c and 40g formed in the base chip 2a communicate with each other.

Therefore, the fluid flowing from the tubes 7a to 7c toward the device 1a flows into the first fluid inlet channel 10a to the third fluid inlet channel 10c (Fig. 13) through the terminal portions 40a to 40c. In addition, the fluid flowing through the mixing channel 20 (Fig. 13) flows out into the tube 7g through the terminal portion 40g.

The main channel 11a of the first fluid inlet channel 10a is formed at the center of the base chip 2a in the Y-axis direction, and is located between the main channel 11b of the second fluid inlet channel 10b and the main channel 11c of the third fluid inlet channel 10c. The main channel 11b of the second fluid inlet channel 10b and the main channel 11c of the third fluid inlet channel 10c are located on both sides (outside) of the main channel 11a of the first fluid inlet channel 10a in the Y-axis direction. The main channel 11b of the second fluid inlet channel 10b and the main channel 11c of the third fluid inlet channel 10c have a shape that is symmetric (mirror-symmetric) with respect to the main channel 11a of the first fluid inlet channel 10a.

Each of the main fluid inlet channels 11a, 11b, and 11c extends linearly along the X-axis; however, the main channel 11a is formed to be longer along the X-axis than the main channels 11b and 11c. In addition, in the present embodiment, the expansion channel 14a of the first fluid inlet channel 10a is formed such that the channel length is longer compared to the expansion channel 14b of the second fluid inlet channel 10b and the expansion channel 14c of the third fluid inlet channel 10c.

The channel resistance from the terminal portion 40b through the first fluid inlet channel 10a to the inlet of the mixing channel 20 can also be made greater than the channel resistance from each of the terminal portions 40a and 40c through the fluid inlet channel 10b or 10c to the inlet of the mixing channel 20. Alternatively, the opposite relationship may be set, or the channel resistances may be set to be the same.

The flow rate of the fluid expelled from each of the channels 11a to 11c into the mixing channel 20 can also be changed. It should be noted that the flow rate of the fluid expelled into the mixing channel 20 can also be changed by changing the fluid pressure applied to each of the terminal portions 40a to 40c on the upstream side. The flow rates of the fluid expelled from the channels 11a to 11c into the mixing channel 20 may be the same or may differ from each other. For example, when the first liquid is a liquid containing phospholipids and the second liquid is a liquid containing a water-soluble material, it is preferable that the flow rate of the second liquid flowing into the mixing channel 20 from the channels 11b and 11c is greater than the flow rate of the first liquid flowing into the mixing channel 20 from the channel 11a, and for example, it is preferable that the flow rate of the second liquid is two times or more, three times or more, five times or more, or eight times more that of the first liquid.

In the present embodiment, the fluid that flows into the mixing channel 20 and is mixed therein can be extracted to the exterior from the terminal portion 40g shown in Fig. 13 through the tube 7g shown in Fig. 12, without passing through the fluid outlet channel 30 (see Fig. 2) of the first embodiment. The microfluidic device 1a of the present embodiment can be attached to, for example, the constant-temperature water bath shown in Fig. 3 in the same manner as in the first embodiment.

Accordingly, the microfluidic device 1a and the fluid flowing through the interiors thereof are maintained at a predetermined temperature. The temperature of the fluid stored in the constant-temperature water bath (the temperature of the microfluidic device 1a (in more detail, the first fluid inlet channel 10a to the third fluid inlet channel 10c and the mixing channel 20), the tubes 7a to 7c and 7g, and the fluid flowing through the interiors thereof is maintained at room temperature or higher (for example, 10°C or higher, preferably 15°C or higher, or 20°C or higher). In addition, the upper limit of the temperature of the fluid in the mixing channel 20 is not particularly limited, but is preferably equal to or lower than the temperature at which the first liquid undergoes degradation, and for example, when the first liquid contains a lipid as a material, the upper limit of the temperature of the fluid is preferably 65°C or less.

As shown in Fig. 13, in the method for manufacturing nanoparticles according to the present embodiment, the first liquid containing at least one material is introduced into the mixing channel 20 through the first fluid inlet channel 10a of the microfluidic device 1a, and the second liquid is introduced into the mixing channel 20 through the second fluid inlet channel 10b and the third fluid inlet channel 10c on both sides of the first fluid inlet channel 10a.

The first liquid is a solution containing at least one material. The material contained in the first liquid is the same as that in the first embodiment. The second liquid is, for example, an aqueous solvent, and specifically, is the same as that in the first embodiment. The first liquid is introduced into the microfluidic device 1a through the tube 7b, for example, by a syringe pump. The second liquid is introduced into the microfluidic device 1a through the tubes 7a and 7c, for example, by a syringe pump.

The first liquid flows into the first fluid inlet channel 10a through the terminal portion 40b. The second liquid flows into the second fluid inlet channel 10b and the third fluid inlet channel 10c through the terminal portions 40a and 40c. In addition, the first liquid is introduced into the mixing channel 20 through the first fluid inlet channel 10a. In addition, the second liquid is introduced into the mixing channel 20 through the second fluid inlet channel 10b and the third fluid inlet channel 10c.

Further, the first liquid and the second liquid are mixed in the mixing channel 20, and are discharged from the mixing channel 20. In more detail, all of the fluid flowing through the mixing channel 20 flows into the tube 7g through the terminal portion 40g. The fluid that has flowed into the tube 7g is stored in a container such as a test tube.

In the present embodiment, the Reynolds number in the mixing channel 20 is set to a predetermined value or greater. When the Reynolds number in the mixing channel 20 is set to the first or second predetermined value, the flow of the fluid as shown in Fig. 4(a) or 4(b) occurs in the mixing channel 20, similar to the first embodiment. In the present embodiment, the same operational effects as those in the above-described first embodiment can be achieved, and further, the following operational effects can also be expected.

In the present embodiment, it is confirmed that the Reynolds number can be further increased compared to the first embodiment. The Reynolds number may be 600 or greater, 800 or greater, 1000 or greater, or 2000 or greater, and even at approximately room temperature, rapid and uniform mixing of the first liquid and the second liquid is promoted, so that fine nanoparticles can be readily manufactured. It should be noted that the upper limit of the Reynolds number is not particularly limited, but is 6000 or less, preferably 4000 or less, or 2400 or less from the viewpoint of the pressure resistance of the microfluidic device 1a, the connectors 70a to 70c and 70g, or the like.

Third embodiment

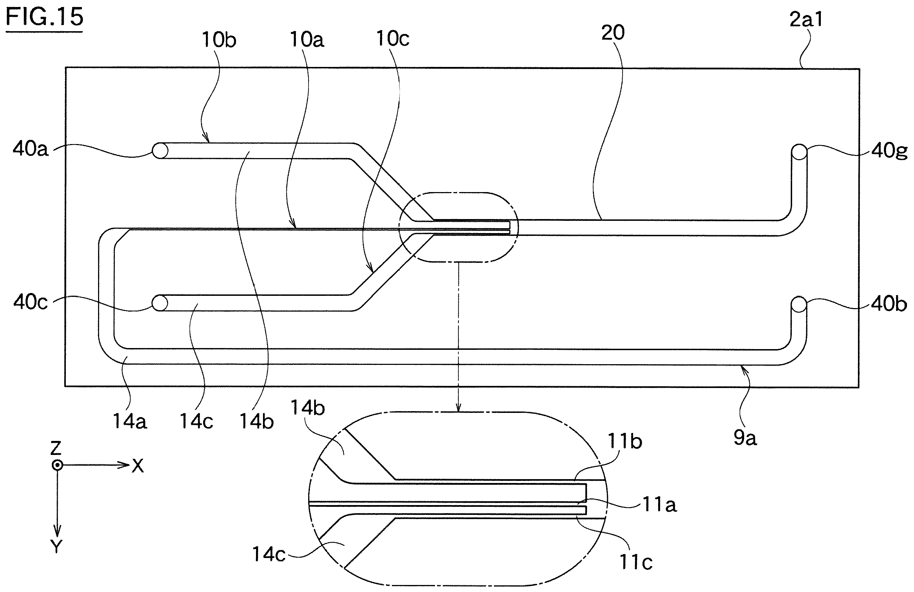

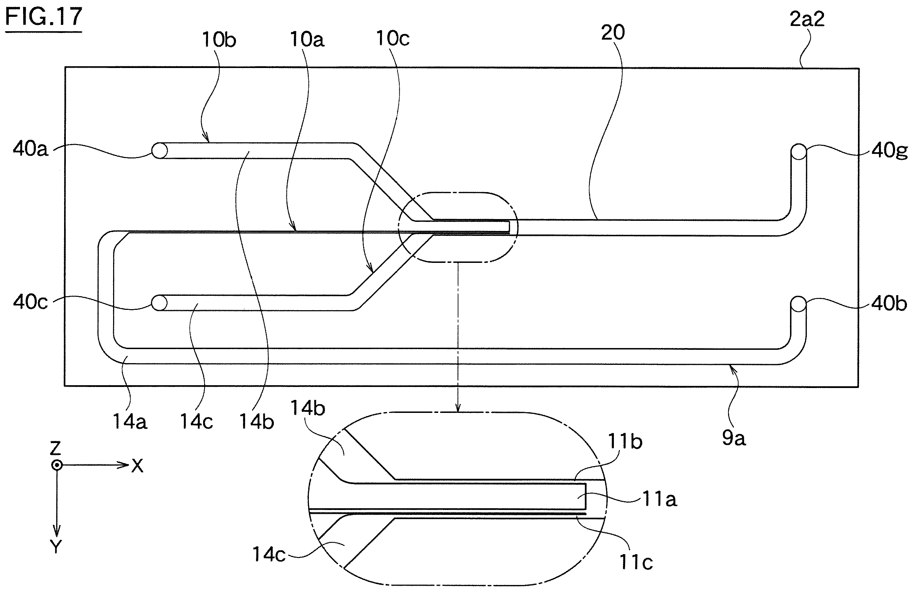

In the present embodiment, as shown in Fig. 15 or 17, in a base chip 2a1 or 2a2, the main fluid inlet channel 11a located between the two main fluid inlet channel 11b and main fluid inlet channel 11c located at both ends along the Y-axis is offset from the centerline of the mixing channel such that the main fluid inlet channel 11a approaches one of the main fluid inlet channel 11b and the main fluid inlet channel 11c. The difference between the embodiment shown in Fig. 15 and the embodiment shown in Fig. 17 is that, in the embodiment shown in Fig. 17, the amount of offset from the centerline of the mixing channel is greater compared to the embodiment shown in Fig. 15.

In the present embodiment, the other configurations are the same as those in the above-described second embodiment, and the same operational effects are achieved. The description of portions common to the above-described embodiments is partially omitted, and in the following description, portions that differ from the above-described embodiments will be mainly described.

In the above-described embodiments, the main fluid inlet channel 11a of the first fluid inlet channel 10a is located on the extension of the centerline passing through the center of the mixing channel 20 along the Y-axis and extending along the X-axis. In the present embodiment, the main fluid inlet channel 11a of the first fluid inlet channel 10a is offset from the centerline of the mixing channel 20 such that the main fluid inlet channel 11a approaches one of the main fluid inlet channels 11b and 11c.

In the present embodiment as well, the flow of the first liquid immediately after entering the mixing channel 20 through the main fluid inlet channel 11a of the first fluid inlet channel 10a becomes asymmetric with respect to the centerline of the main fluid inlet channel 11a such that the flow of the first liquid is biased toward one of the two side walls of the mixing channel 20. In the above-described embodiments, the direction of the flow bias of the first liquid immediately after entering the mixing channel 20 can be toward one of the two side walls of the mixing channel 20. However, in the present embodiment, the direction of the flow bias of the first liquid immediately after entering the mixing channel 20 is often toward the side wall of the mixing channel 20 offset from the centerline and located close to the main fluid inlet channel 11a. The other configurations and the operational effects are the same as those in the above-described embodiments.

It should be noted that, in the above-described first to third embodiments, the mixing channel 20 is a channel with a rectangular cross-section; however, the embodiments of the present invention are not limited thereto. For example, the configuration is not particularly limited as long as the configuration is devised such that the flow of the first liquid in the mixing channel is a flow in a sealed space and becomes asymmetric such that the flow of the first liquid is biased toward one of the two side walls of the mixing channel. For example, the transverse cross-section of the mixing channel 20 is not limited to a rectangular shape, but may have a quadrilateral shape other than a rectangular shape, a polygonal shape with three or more sides, a circular shape, an elliptical shape, any other irregular cross-sectional shape, or the like.

[Examples]

Hereinafter, the present invention will be further described based on detailed examples; however, the present invention is not limited to these examples.

Example 1

In fabricating the microfluidic device 1 shown in Figs. 1 and 2, a silicon wafer was processed using a deep reactive ion etching apparatus (Sumitomo Precision Products Corporation: MUC-21ASE-SRE) to fabricate a mold for the microchannel pattern (the groove 9) of the base chip 2a. The depth of the groove 9 was set to 0.2 mm. The lateral width and the length of each of the main fluid inlet channels 11a to 11c were set to 0.1 mm and 5 mm, respectively. The spacing (the center-to-center distance) between the main fluid inlet channels 11a to 11c was set to 0.45 mm. The lateral width and the length of the mixing channel 20 were set to 1 mm and 10 mm, respectively. The lateral width and the length of each of the main fluid outlet channels 31a to 31e were set to 0.1 mm and 6 mm, respectively. The spacing (the center-to-center distance) between the main fluid outlet channels 31a to 31e was set to 0.2 mm.

The base chip 2a, onto which the microchannel pattern was transferred, and the cover chip 2b were fabricated by injection molding at Richell Corporation. In addition, the base chip 2a and the cover chip 2b were superimposed on top of each other, and were joined by pressing at 80°C for one hour. In the manner described above, the microfluidic device 1 shown in Figs. 1 and 2 was fabricated.

As shown in Fig. 1, the connectors 70a to 70f were attached to the through-holes 3a to 3f of the cover chip 2b, and the microfluidic device 1 was clamped and fixed from above and below using the clamping plates 4a and 4b. In addition, the tubes 7a to 7e were connected to the connectors 70a to 70f. PTFE tubes, each having an inner diameter of 0.5 mm and a length of 1.5 mm, were used as the tubes 7a to 7c.

In addition, PTFE tubes, each having an inner diameter of 1 mm and a length of 25 cm, were used as the tubes 7d and 7e. However, the tube 7d includes the branched tubes 7d1 and 7d2. Furthermore, the tubes 7a to 7c were connected to syringe pumps (Chemyx Corporation: Fusion 200).

As shown in Figs. 1 and 2, using the fabricated microfluidic device 1, the first liquid was introduced into the mixing channel 20 through the tube 7b via the first fluid inlet channel 10a, the second liquid was introduced into the mixing channel 20 through the tube 7a via the second fluid inlet channel 10b, the second liquid was introduced into the mixing channel 20 through the tube 7c via the third fluid inlet channel 10c, and the first liquid and the second liquid were mixed. Ethanol was used as the first liquid, and pure water was used as the second liquid. In addition, the fluid discharged from the mixing channel 20 through the tubes 7d and 7e was collected in a container.

In introducing the first liquid and the second liquid into the microfluidic device 1, the flow rate ratio of the second liquid (the sum of the second liquid flowing through the second fluid inlet channel 10b and the second liquid flowing through the third fluid inlet channel 10c) to the first liquid was set to 10:1, the flow rate ratio of the second liquid flowing through the second fluid inlet channel 10b to the second liquid flowing through the third fluid inlet channel 10c was set to 10:10, the temperature of the mixing channel 20 was set to approximately 20°C (room temperature), the total flow rate of the first liquid and the second liquid (the flow rate of the fluid flowing through the mixing channel 20) was set to 7.7 mL/min, and the Reynolds number in the mixing channel 20 was set to 151.

However, the Reynolds number Re in the mixing channel 20 was calculated based on Equation (1) described above. In Equation (1), w was set to 1 mm, and h was set to 0.2 mm. For $\rho$ and $\mu$, values corresponding to when ethanol (the first liquid) and pure water (the second liquid) were completely mixed (literature values or calculated values based on the literature values) were used.

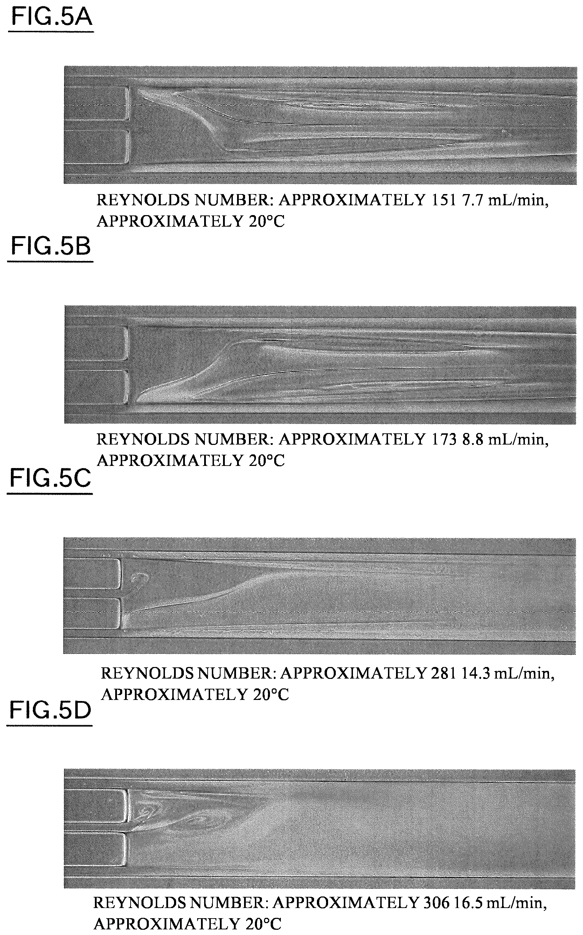

The state of flow of the fluid flowing through the mixing channel 20 was observed under the above conditions. The observation and imaging of the mixing channel 20 were performed using an inverted phase contrast microscope (Olympus Corporation: CKX-53) to which a digital video camera (Sony Corporation: HDR-PJ630) was attached. The result is shown in Fig. 5(a). As shown in Fig. 5(a), the flow of the first liquid immediately after entering the mixing channel 20 through the first fluid inlet channel 10a became asymmetric such that the flow of the first liquid was biased toward one of the two side walls of the mixing channel 20.

This result showed that the first liquid and the second liquid were more easily mixed at the initial stage where the first liquid and the second liquid flowed into the mixing channel 20; therefore, the mixing efficiency of the first liquid and the second liquid was improved. It should be noted that, in Fig. 5(a) and subsequent figures, in order to prevent the drawings from becoming overly complex, the illustration of the reference signs for the first fluid inlet channel 10a, the second fluid inlet channel 10b, the third fluid inlet channel 10c, and the mixing channel 20 and the illustration of the XYZ coordinate axes are omitted.

Example 2

The total flow rate of the first liquid and the second liquid was set to 8.8 mL/min, and the Reynolds number in the mixing channel 20 was set to 173. The other experimental conditions were set to be the same as those in Example 1. The result is shown in Fig. 5(b). As shown in Fig. 5(b), the flow of the first liquid immediately after entering the mixing channel 20 through the first fluid inlet channel 10a became asymmetric such that the flow of the first liquid was biased toward one of the two side walls of the mixing channel 20.

This result showed that the same effects as those in Example 1 were obtained. It should be noted that the direction of the bias of the first liquid was a direction different from the direction of the bias of the first liquid shown in Fig. 5(a) (opposite direction). However, the direction of the bias of the first liquid might be the same as the direction of the bias of the first liquid shown in Fig. 5(a).

Example 3

The total flow rate of the first liquid and the second liquid was set to 14.3 mL/min, and the Reynolds number in the mixing channel 20 was set to 281. The other experimental conditions were set to be the same as those in Example 1. The result is shown in Fig. 5(c). As shown in Fig. 5(c), the flow of the first liquid immediately after entering the mixing channel 20 through the first fluid inlet channel 10a became asymmetric such that the flow of the first liquid was biased toward one of the two side walls of the mixing channel 20. In addition, immediately after the first liquid has entered the mixing channel 20 through the first fluid inlet channel 10a, an ultra-microvortex was observed.

These results showed that the first liquid and the second liquid were more easily mixed at the initial stage where the first liquid and the second liquid flowed into the mixing channel 20; therefore, the mixing efficiency of the first liquid and the second liquid was improved. Additionally, the boundary between the microvortex region 50 and the regions on both sides thereof (the regions mainly containing the second liquid) disappeared (became indistinct). This result showed that the mixing of the first liquid and the second liquid was also promoted between these regions; therefore, the mixing efficiency of the first liquid and the second liquid was further improved.

It should be noted that the microvortex also becomes indistinct in the microvortex region 50 shown in Fig. 5(c). The region where the microvortex becomes indistinct and the boundary between the microvortex region 50 and the regions on both sides thereof (the regions mainly containing the second liquid) also becomes indistinct is hereinafter referred to as a uniform mixing region.

Example 4

The total flow rate of the first liquid and the second liquid was set to 16.5 mL/min, the Reynolds number in the mixing channel 20 was set to 306, and the flow rate ratio of the second liquid to the first liquid was set to 8:1. The other experimental conditions were set to be the same as those in Example 1. The result is shown in Fig. 5(d).

As shown in Fig. 5(d), the flow of the first liquid immediately after entering the mixing channel 20 through the first fluid inlet channel 10a became asymmetric such that the flow of the first liquid was more sharply biased toward one of the two side walls of the mixing channel 20. In addition, immediately after the first liquid has entered the mixing channel 20 through the first fluid inlet channel 10a, two ultra-microvortices were observed to be aligned approximately in the X-axis direction.

Further, the position where the boundary between the microvortex region 50 and the regions on both sides thereof (the regions mainly containing the second liquid) disappeared (became indistinct) moved to the upstream side of the mixing channel 20. In addition, it was confirmed that the uniform mixing region was obtained from the upstream side. The above results showed that the mixing of the first liquid and the second liquid proceeded at an earlier stage (further upstream in the mixing channel 20).

Example 5

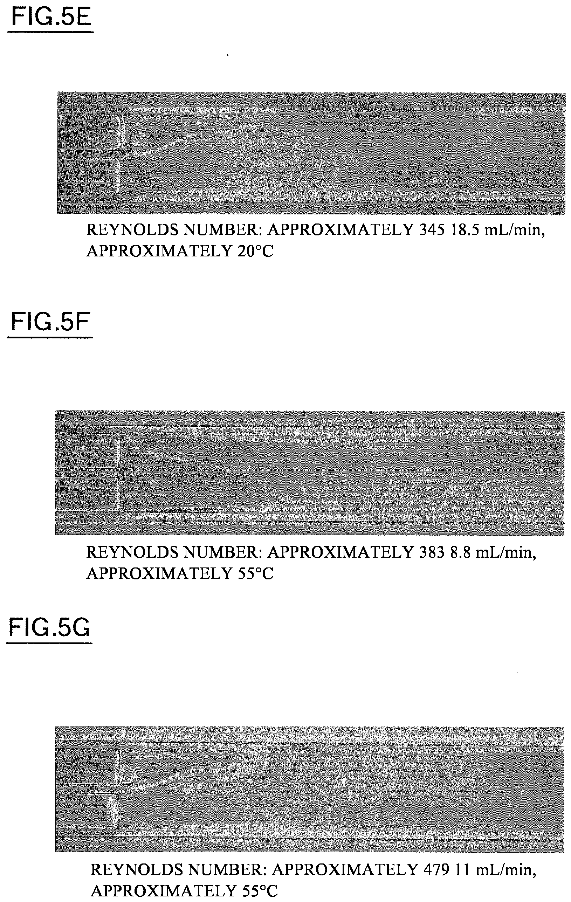

The total flow rate of the first liquid and the second liquid was set to 18.5 mL/min, and the Reynolds number in the mixing channel 20 was set to 345. The other experimental conditions were set to be the same as those in Example 4. The result is shown in Fig. 5(e). As shown in Fig. 5(e), the flow of the first liquid immediately after entering the mixing channel 20 through the first fluid inlet channel 10a became asymmetric such that the flow of the first liquid was more sharply biased toward one of the two side walls of the mixing channel 20.

In addition, immediately after the first liquid has entered the mixing channel 20 through the first fluid inlet channel 10a, an ultra-microvortex was observed. Further, the position where the boundary between the microvortex region 50 and the regions on both sides thereof (the regions mainly containing the second liquid) disappeared (became indistinct) moved to the upstream side of the mixing channel 20. In addition, it was confirmed that the uniform mixing region was obtained from the upstream side. The above results showed that the mixing of the first liquid and the second liquid proceeded at an earlier stage (further upstream in the mixing channel 20).

Example 6

The total flow rate of the first liquid and the second liquid was set to 8.8 mL/min, and the Reynolds number in the mixing channel 20 was set to 383. In addition, as shown in Fig. 3, the microfluidic device 1, together with the tubes 7a to 7e, was immersed in a constant-temperature water bath heated to 55°C, to increase the temperature of the mixing channel 20 to 55°C. The observation and imaging of the mixing channel 20 were performed with the microfluidic device 1 removed from the constant-temperature water bath. The other experimental conditions were set to be the same as those in Example 1.

The result is shown in Fig. 5(f). As shown in Fig. 5(f), the flow of the first liquid immediately after entering the mixing channel 20 through the first fluid inlet channel 10a became asymmetric such that the flow of the first liquid was biased toward one of the two side walls of the mixing channel 20. In addition, the boundary between the microvortex region 50 and the regions on both sides thereof (the regions mainly containing the second liquid) disappeared (became indistinct). The above results showed that the mixing of the first liquid and the second liquid was also promoted between these regions; therefore, the mixing efficiency of the first liquid and the second liquid was further improved.

Example 7

The total flow rate of the first liquid and the second liquid was set to 11 mL/min, and the Reynolds number in the mixing channel 20 was set to 479. The other experimental conditions were set to be the same as those in Example 6. The result is shown in Fig. 5(g). As shown in Fig. 5(g), the flow of the first liquid immediately after entering the mixing channel 20 through the first fluid inlet channel 10a became asymmetric such that the flow of the first liquid was more sharply biased toward one of the two side walls of the mixing channel 20.

In addition, immediately after the first liquid has entered the mixing channel 20 through the first fluid inlet channel 10a, two ultra-microvortices were observed to be aligned approximately in the X-axis direction. Further, the position where the boundary between the microvortex region 50 and the regions on both sides thereof (the regions mainly containing the second liquid) disappeared (became indistinct) moved to the upstream side of the mixing channel 20. In addition, it was confirmed that the uniform mixing region was obtained from the upstream side. The above results showed that the mixing of the first liquid and the second liquid proceeded at an earlier stage (further upstream in the mixing channel 20).

Example 8

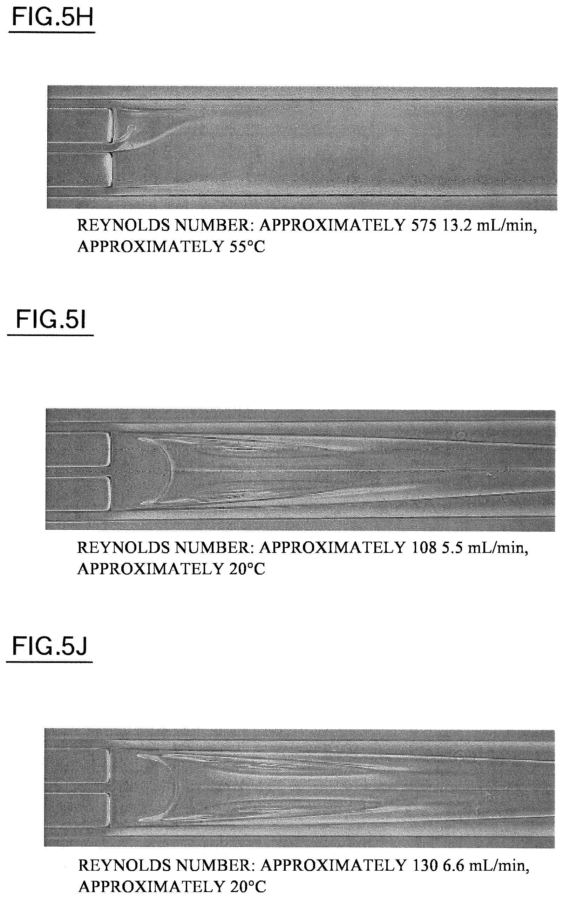

The total flow rate of the first liquid and the second liquid was set to 13.2 mL/min, and the Reynolds number in the mixing channel 20 was set to 575. The other experimental conditions were set to be the same as those in Example 6. The result is shown in Fig. 5(h). As shown in Fig. 5(h), the flow of the first liquid immediately after entering the mixing channel 20 through the first fluid inlet channel 10a became asymmetric such that the flow of the first liquid was more sharply biased toward one of the two side walls of the mixing channel 20.

In addition, immediately after the first liquid has entered the mixing channel 20 through the first fluid inlet channel 10a, an ultra-microvortex was observed. Further, the position where the boundary between the microvortex region 50 and the regions on both sides thereof (the regions mainly containing the second liquid) disappeared (became indistinct) moved further upstream in the mixing channel 20. In addition, it was confirmed that the uniform mixing region was obtained from the upstream side. The above results showed that the mixing of the first liquid and the second liquid proceeded at an earlier stage (further upstream in the mixing channel 20).

Comparative Example 1

The total flow rate of the first liquid and the second liquid was set to 5.5 mL/min, and the Reynolds number in the mixing channel 20 was set to 108. The other experimental conditions were set to be the same as those in Example 1. The result is shown in Fig. 5(i). As shown in Fig. 5(i), the flow of the first liquid immediately after entering the mixing channel 20 through the first fluid inlet channel 10a became symmetric with respect to the first fluid inlet channel 10a.

In addition, a microvortex symmetric with respect to the first fluid inlet channel 10a was formed in the mixing channel 20. The above results showed that the mixing efficiency of the first liquid and the second liquid might not be sufficient, and it might be difficult to realize rapid and uniform mixing of the first liquid and the second liquid.

Comparative Example 2

The total flow rate of the first liquid and the second liquid was set to 6.6 mL/min, and the Reynolds number in the mixing channel 20 was set to 130. The other experimental conditions were set to be the same as those in Example 1. The result is shown in Fig. 5(j). As shown in Fig. 5(j), the flow of the first liquid immediately after entering the mixing channel 20 through the first fluid inlet channel 10a became symmetric with respect to the first fluid inlet channel 10a.

In addition, a microvortex symmetric with respect to the first fluid inlet channel 10a was formed in the mixing channel 20. The above results showed that the mixing efficiency of the first liquid and the second liquid might not be sufficient, and it might be difficult to realize rapid and uniform mixing of the first liquid and the second liquid.

Example 9

The flow rate ratio of the second liquid flowing through the second fluid inlet channel 10b to the second liquid flowing through the third fluid inlet channel 10c was set to 10:7 (more accurately, 10:6.77), the flow rate ratio of the second liquid (the sum of the second liquid flowing through the second fluid inlet channel 10b and the second liquid flowing through the third fluid inlet channel 10c) to the first liquid was set to 8.5:1, the total flow rate of the first liquid and the second liquid was set to 9.4 mL/min, and the Reynolds number in the mixing channel 20 was set to 175. The other experimental conditions were set to be the same as those in Example 1.

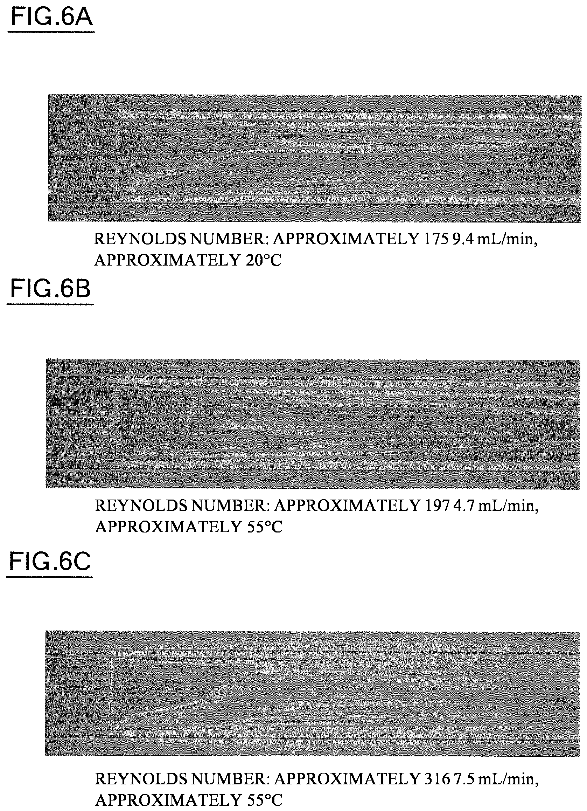

The result is shown in Fig. 6(a). As shown in Fig. 6(a), the flow of the first liquid immediately after entering the mixing channel 20 through the first fluid inlet channel 10a became asymmetric such that the flow of the first liquid was biased toward one of the two side walls of the mixing channel 20. This result showed that the first liquid and the second liquid were more easily mixed at the initial stage where the first liquid and the second liquid flowed into the mixing channel 20; therefore, the mixing efficiency of the first liquid and the second liquid was improved.

Example 10

The total flow rate of the first liquid and the second liquid was set to 4.7 mL/min, and the Reynolds number in the mixing channel 20 was set to 197. In addition, the temperature of the mixing channel 20 was set to 55°C. The other experimental conditions were set to be the same as those in Example 9. The result is shown in Fig. 6(b). As shown in Fig. 6(b), the flow of the first liquid immediately after entering the mixing channel 20 through the first fluid inlet channel 10a became asymmetric such that the flow of the first liquid was biased toward one of the two side walls of the mixing channel 20. This result showed that the same effects as those in Example 9 were obtained.

Example 11

The total flow rate of the first liquid and the second liquid was set to 7.5 mL/min, and the Reynolds number in the mixing channel 20 was set to 316. The other experimental conditions were set to be the same as those in Example 10. The result is shown in Fig. 6(c). As shown in Fig. 6(c), the flow of the first liquid immediately after entering the mixing channel 20 through the first fluid inlet channel 10a became asymmetric such that the flow of the first liquid was biased toward one of the two side walls of the mixing channel 20.

In addition, the boundary between the microvortex region 50 and the regions on both sides thereof (the regions mainly containing the second liquid) disappeared (became indistinct). The above results showed that the mixing of the first liquid and the second liquid was also promoted between these regions; therefore, the mixing efficiency of the first liquid and the second liquid was further improved.

Example 12

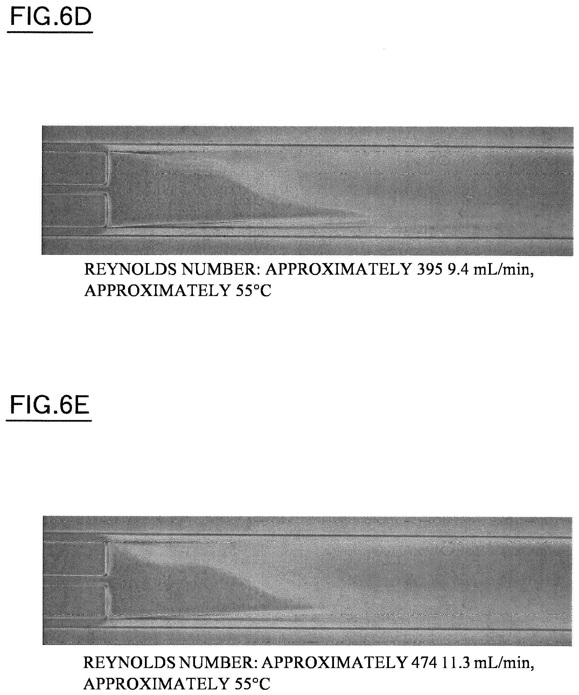

The total flow rate of the first liquid and the second liquid was set to 9.4 mL/min, and the Reynolds number in the mixing channel 20 was set to 395. The other experimental conditions were set to be the same as those in Example 10. The result is shown in Fig. 6(d). As shown in Fig. 6(d), the flow of the first liquid immediately after entering the mixing channel 20 through the first fluid inlet channel 10a became asymmetric such that the flow of the first liquid was biased toward one of the two side walls of the mixing channel 20.

In addition, the position where the boundary between the microvortex region 50 and the regions on both sides thereof (the regions mainly containing the second liquid) disappeared (became indistinct) moved further upstream in the mixing channel 20. In addition, it was confirmed that the uniform mixing region was obtained from the upstream side. The above results showed that the mixing of the first liquid and the second liquid proceeded at an earlier stage (further upstream in the mixing channel 20).

Example 13

The total flow rate of the first liquid and the second liquid was set to 11.3 mL/min, and the Reynolds number in the mixing channel 20 was set to 474. The other experimental conditions were set to be the same as those in Example 10. The result is shown in Fig. 6(e). As shown in Fig. 6(e), the flow of the first liquid immediately after entering the mixing channel 20 through the first fluid inlet channel 10a became asymmetric such that the flow of the first liquid was biased toward one of the two side walls of the mixing channel 20.

In addition, the position where the boundary between the microvortex region 50 and the regions on both sides thereof (the regions mainly containing the second liquid) disappeared (became indistinct) moved further upstream in the mixing channel 20. In addition, it was confirmed that the uniform mixing region was obtained from the upstream side. The above results showed that the mixing of the first liquid and the second liquid proceeded at an earlier stage (upstream in the mixing channel 20).

Example 14

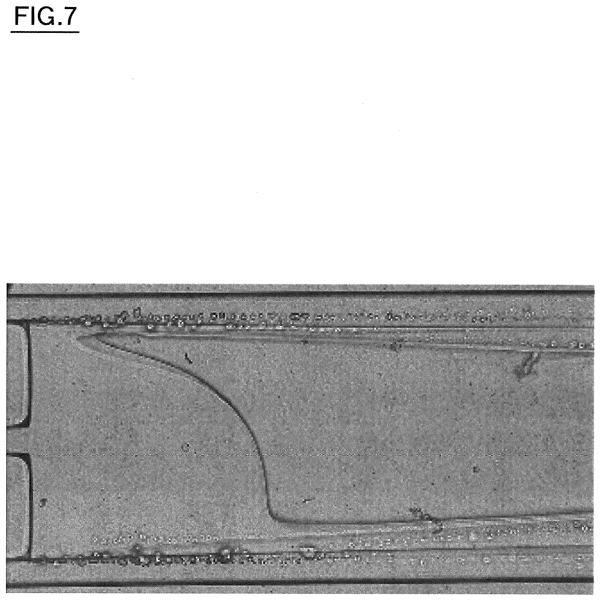

The total flow rate of the first liquid and the second liquid was set to 5.5 mL/min, and the Reynolds number in the mixing channel 20 was set to 240. In addition, the temperature of the mixing channel 20 was set to 55°C. The other experimental conditions were set to be the same as those in Example 1. The result is shown in Fig. 7. As shown in Fig. 7, when the temperature of the mixing channel 20 was set to 55°C, unlike Comparative Example 1 in which the temperature of the mixing channel 20 was set to 20°C, the flow of the first liquid immediately after entering the mixing channel 20 through the first fluid inlet channel 10a became asymmetric such that the flow of the first liquid was biased toward one of the two side walls of the mixing channel 20.

This result showed that, when the total flow rate of the first liquid and the second liquid was substantially the same, the increase in the temperature of the mixing channel 20 to 55°C caused the first liquid and the second liquid to be more easily mixed at the initial stage where the first liquid and the second liquid flowed into the mixing channel 20; therefore, the mixing efficiency of the first liquid and the second liquid was improved.

Example 15

As will be described below, reconstituted high-density lipoprotein (rHDL) was produced by mixing the first liquid containing distearoylphosphatidylcholine (DSPC) as a phospholipid and the second liquid containing apolipoprotein A-I (apoA-I) as a protein, using the microfluidic device 1.

A 5 mg/mL lipid solution in which DSPC was dissolved in ethanol was used as the first liquid. A 200 $\mu$g/mL protein solution in which apolipoprotein A-I was dissolved in phosphate-buffered saline (PBS) containing 2M Urea was used as the second liquid. However, a variant in which the N-terminal 43 amino acids were deleted was used as the apolipoprotein A-I. The density of the second liquid (2M Urea solution) at 25°C was approximately 1.03, and the viscosity was approximately 1.09. In addition, as shown in Fig. 3, the microfluidic device 1, together with the tubes 7a to 7e, was immersed in a constant-temperature water bath heated to 55°C, to increase the temperature of the mixing channel 20 to 55°C that is the phase transition temperature of DSPC.

As shown in Figs. 1 to 3, the first liquid was introduced into the mixing channel 20 through the tube 7b via the first fluid inlet channel 10a using the fabricated microfluidic device 1 (the same microfluidic device 1 as that in Example 1). In addition, the second liquid was introduced into the mixing channel 20 through the tube 7a via the second fluid inlet channel 10b. In addition, the second liquid was introduced into the mixing channel 20 through the tube 7c via the third fluid inlet channel 10c. Accordingly, the first liquid and the second liquid were mixed in the mixing channel 20. Then, the fluid discharged from the mixing channel 20 through the tubes 7d and 7e was collected in a container.

In introducing the first liquid and the second liquid into the microfluidic device 1, the flow rate ratio of the second liquid to the first liquid was set to 10:1, the flow rate ratio of the second liquid flowing through the second fluid inlet channel 10b to the second liquid flowing through the third fluid inlet channel 10c was set to 10:10, and the temperature of the mixing channel 20 was set to approximately 55°C. The total flow rate of the first liquid and the second liquid was set to each of 5.5 mL/min, 7.7 mL/min, 8.8 mL/min, 11 mL/min, and 13.2 mL/min.

It should be noted that, when the total flow rate of the first liquid and the second liquid is 5.5 mL/min, the Reynolds number in the mixing channel 20 is 228. In addition, when the total flow rate of the first liquid and the second liquid is 7.7 mL/min, the Reynolds number in the mixing channel 20 is 319. When the total flow rate of the first liquid and the second liquid is 8.8 mL/min, the Reynolds number in the mixing channel 20 is 364. When the total flow rate of the first liquid and the second liquid is 11 mL/min, the Reynolds number in the mixing channel 20 is 456. When the total flow rate of the first liquid and the second liquid is 13.2 mL/min, the Reynolds number in the mixing channel 20 is 547.

The collected solution was dialyzed against 2L of PBS at room temperature for two hours using a dialysis membrane having a molecular weight cutoff of 3 kDa (Spectrum Chemical Corporation: Spectra/Por 6 Pre-Wetted RC dialysis membrane). The obtained sample was centrifuged at 20000 x g for 15 minutes at 24°C to obtain a supernatant. The supernatant was purified and analyzed by size exclusion chromatography (SEC). The purification and analysis were performed by flowing the supernatant through a column (GE Healthcare: HiLoad 16/60 Superdex 200 prepgrade) attached to a high-performance liquid chromatograph (Shimadzu Corporation: Prominence HPLC System), using PBS at a flow rate of 1 mL/min at room temperature, and measuring the absorbance of the eluate from the column at 280 nm.

The average hydrodynamic diameter of the rHDL sample was measured using a particle size distribution analyzer (MicrotracBEL Corporation: Nanotrac UPA-UT151). In addition, the protein content of the rHDL was analyzed using a DC protein assay kit manufactured by Bio-Rad Laboratories and a multi-mode plate reader (BioTek Instrument: Synergy HTX Multi-Mode Microplate Reader) and using bovine serum albumin manufactured by Thermo Fisher Scientific as a standard substance.

In addition, the lipid content of the rHDL was analyzed using a phospholipid assay kit (FUJIFILM Wako Pure Chemical Corporation: Phospholipid C assay kit) and a multi-mode plate reader (BioTek Instrument: Synergy HTX Multi-Mode Microplate Reader). In addition, the surface structure of the particles was observed using an atomic force microscope (AFM). The AFM observation was performed with a cantilever (Olympus Corporation: BL-AC40TS) attached to an SPM-9700HT manufactured by Shimadzu Corporation. The rHDL sample was dropped onto a mica surface, and was allowed to stand for four minutes. Next, the floating sample was rinsed with pure water, and the rHDL sample adsorbed onto the mica surface was observed in pure water using an AFM.

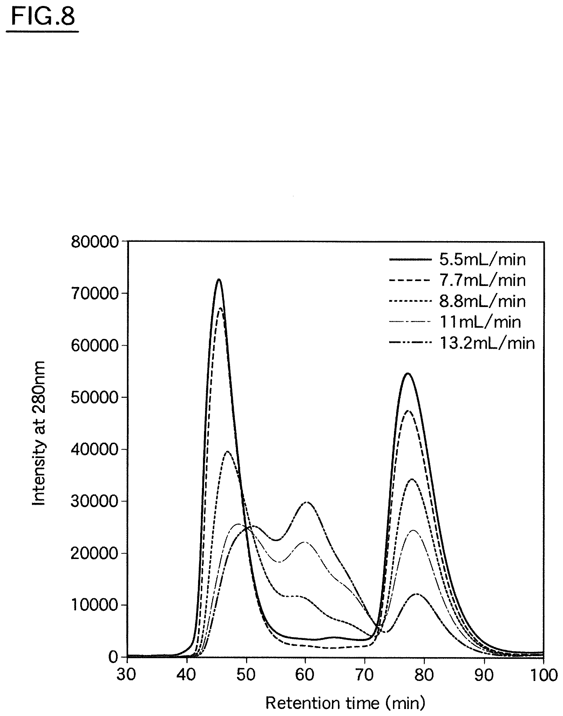

Fig. 8 shows the results of size exclusion chromatography at each flow rate when the total flow rate (TFR) of the first liquid and the second liquid was set to each of 5.5 mL/min, 7.7 mL/min, 8.8 mL/min, 11 mL/min, and 13.2 mL/min. In addition, Table 1 shows the average hydrodynamic diameter, the yield, and the proportion of free protein (protein remaining without binding to lipid) for each flow rate.

In addition, Table 2 shows the average hydrodynamic diameter, the yield, and the molar ratio of lipid to protein (lipid/protein) for each peak (a peak around a retention time of 50 minutes and a peak around a retention time of 60 minutes) when the total flow rate of the first liquid and the second liquid was set to 13.2 mL/min. It should be noted that the results when the total flow rate of the first liquid and the second liquid was set to 13.2 mL/min are the average values of three experiments (n = 3).

[Table 1]

| Total flow rate [mL/min] | Average hydrodynamic diameter [nm] | Yield (on protein basis) [%] | Free protein [%] |
|---|---|---|---|
| 5.5 | 33.7 | 25 | 69 |
| 7.7 | 28.2 | 31 | 59 |
| 11 | 15.4 | 78 | 31 |
| 13.2 | 14.4±0.2 | 79±6 | 17±4 |

[Table 2]

| Peak | Average hydrodynamic diameter [nm] | Yield (on protein basis) [%] | Molar ratio of lipid to protein [%] |
|---|---|---|---|
| Peak 1 around a retention time of 50 minutes (43 to 55 minutes) | 15.4±1.2 | 31±2 | 108±6 |
| Peak 2 around a retention time of 60 minutes (55 to 70 minutes) | 9.8±0.8 | 48±4 | 49±1 |

As shown in Fig. 8, it was confirmed that, as the total flow rate of the first liquid and the second liquid increased, the number of the particles having a larger particle size represented by the peak around a retention time of 45 minutes decreased, whereas the number of the particles having a smaller particle size represented by the peak around a retention time of 60 minutes increased. In addition, as shown in Table 1, it was confirmed that, as the total flow rate of the first liquid and the second liquid increased, the average hydrodynamic diameter decreased. In addition, as shown in Table 1, when the total flow rate of the first liquid and the second liquid was 13.2 mL/min, the average hydrodynamic diameter was 14.4 ± 0.2 nm.

In addition, as shown in Table 2, when the total flow rate of the first liquid and the second liquid was 13.2 mL/min, the average hydrodynamic diameter around a retention time of 50 minutes (a retention time of 43 to 55 minutes) was 15.4 ± 1.2 nm. Meanwhile, the average hydrodynamic diameter around a retention time of 60 minutes (a retention time of 55 to 70 minutes) was 9.8 ± 0.8 nm. In addition, it was confirmed by an AFM that the particles having a smaller particle sizes represented by the peaks around retention times of 50 minutes and 60 minutes had a disk-shaped structure.

From the above results, it was confirmed that the particles having a smaller particle size represented by the peaks around retention times of 50 minutes and 60 minutes were rHDL using DSPC as a lipid (DSPC-rHDL). In this way, it was confirmed that in the method for manufacturing nanoparticles according to the present invention, the reconstitution of DSPC-rHDL that was difficult to manufacture using conventional methods for manufacturing nanoparticles using microfluidic devices is enabled.

In addition, it was confirmed that the method for manufacturing nanoparticles according to the present invention was suitable for manufacturing fine nanoparticles having a particle size of around 10 nm. In addition, it is considered that, in the method for manufacturing nanoparticles according to the present invention, fine disk-shaped fragments (small pieces of a lipid bilayer) having a particle size of around 10 nm are generated, and DSPC-rHDL is generated by the rapid binding of apolipoprotein A-I to such disk-shaped fragments.

In addition, as shown in Fig. 8, it was confirmed that, as the total flow rate of the first liquid and the second liquid increased, the number of the particles having a smaller particle size represented by the peak around a retention time of 77 minutes decreased. In addition, it was confirmed that the particles having a smaller particle size represented by the peak around a retention time of 77 minutes were free protein. In addition, as shown in Table 1, it was confirmed that, as the total flow rate of the first liquid and the second liquid increased, the yield increased, whereas the proportion of free protein decreased. Accordingly, it was confirmed that, in the method for manufacturing nanoparticles according to the present invention, the mixing efficiency of the first liquid and the second liquid was improved, and rapid and uniform mixing of the first liquid and the second liquid was promoted.

Example 16

The generation efficiency of DSPC-rHDL was evaluated when the temperature of the mixing channel 20 was set to approximately 45°C and approximately 55°C in the following manner. A 2.5 mg/mL lipid solution in which DSPC was dissolved in ethanol was produced as the first liquid. A 100 $\mu$g/mL protein solution in which apolipoprotein A-I was dissolved in PBS containing 2M Urea was produced as the second liquid.

However, a variant in which the N-terminal 43 amino acids were deleted was used as the apolipoprotein A-I. The density of the second liquid (2M Urea solution) at 25°C was approximately 1.03, and the viscosity was approximately 1.09. In addition, as shown in Fig. 3, the microfluidic device 1, together with the tubes 7a to 7e, was immersed in a constant-temperature water bath heated to approximately 45°C or approximately 55°C, to increase the temperature of the mixing channel 20 to approximately 45°C or approximately 55°C.

As shown in Figs. 1 to 3, the first liquid was introduced into the mixing channel 20 at a flow rate of 1 mL/min through the tube 7b via the first fluid inlet channel 10a using the fabricated microfluidic device 1 (the same microfluidic device 1 as that in Example 1). In addition, the second liquid was introduced into the mixing channel 20 at a flow rate of 5 mL/min through the tube 7a via the second fluid inlet channel 10b.

In addition, the second liquid was introduced into the mixing channel 20 at a flow rate of 5 mL/min through the tube 7c via the third fluid inlet channel 10c. Accordingly, the first liquid and the second liquid were mixed in the mixing channel 20. Then, the fluid discharged from the mixing channel 20 through the tubes 7d and 7e was collected in a container. When the temperature of the mixing channel 20 was approximately 45°C, the Reynolds number in the mixing channel 20 was 377. When the temperature of the mixing channel 20 was approximately 55°C, the Reynolds number in the mixing channel 20 was 456.

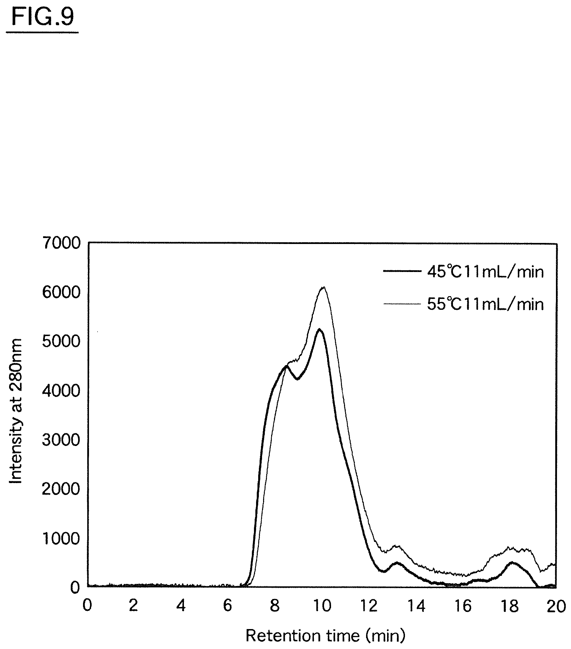

In the same method as that in Example 15, the collected solution was dialyzed against PBS for two hours, and the obtained sample was centrifuged at 20000 x g for 15 minutes at 24°C to obtain a supernatant. Then, the supernatant was subjected to semimicroanalysis by size exclusion chromatography. The semimicroanalysis was performed by flowing the supernatant through a column (Cytiva: Superdex 200 Increase 5/150 GL) attached to a high-performance liquid chromatograph (Shimadzu Corporation: Prominence HPLC System), using PBS at a flow rate of 0.15 mL/min at room temperature, and measuring the absorbance of the eluate from the column at 280 nm. Fig. 9 shows the results of size exclusion chromatography when the temperature of the mixing channel 20 is set to approximately 45°C and approximately 55°C.

As shown in Fig. 9, in both cases where the temperature of the mixing channel 20 was approximately 45°C and approximately 55°C, a large peak was confirmed around a retention time of 10 minutes, and it was confirmed that the particles having a smaller particle size represented by the peak around a retention time of 10 minutes were generated. In addition, it was confirmed that the peak around a retention time of 10 minutes became larger when the temperature of the mixing channel 20 was approximately 55°C than when the temperature of the mixing channel 20 was approximately 45°C.

In the column, the peak around a retention time of 10 minutes is attributed to DSPC-rHDL having a particle size of around 10 nm. Therefore, it was confirmed that DSPC-rHDL having a particle size of around 10 nm was generated in both cases where the temperature of the mixing channel 20 was approximately 45°C and approximately 55°C. In addition, it was confirmed that the generation efficiency of DSPC-rHDL was improved by setting the temperature of the mixing channel 20 to approximately 55°C.

In the method for manufacturing nanoparticles according to the present invention, aggregates of the DSPC contained in the first liquid is made less likely to occur by setting the temperature of the mixing channel 20 to approximately 45°C or higher, so that DSPC-rHDL that is fine nanoparticles can be easily formed.

Example 17

The microfluidic device 1 that was the same as that in Example 1 except for the following points was fabricated. In the microfluidic device 1 of the present example, PTPE tubes, each having an inner diameter of 0.25 mm and a length of 25 cm, were used as the tubes 7d and 7e shown in Figs. 1 and 3. In addition, as shown in Figs. 1 to 3, tube clamps were fixed to the tubes 7d1 and 7d2 and the main fluid outlet channels 31a, 31b, 31d, and 31e were blocked such that the fluid discharged from the mixing channel 20 did not flow through the main fluid outlet channels 31a, 31b, 31d, and 31e.

In addition, a 1 mg/mL lipid solution in which DSPC was dissolved in ethanol was produced as the first liquid. A 40 $\mu$g/mL protein solution in which apolipoprotein A-I was dissolved in PBS containing 2M Urea was produced as the second liquid. However, a variant in which the N-terminal 43 amino acids were deleted was used as the apolipoprotein A-I. The density of the second liquid (2M Urea solution) at 25°C was approximately 1.03, and the viscosity was approximately 1.09. In addition, as shown in Fig. 3, the microfluidic device 1, together with the tubes 7a to 7e, was immersed in a constant-temperature water bath heated to approximately 55°C, to increase the temperature of the mixing channel 20 to approximately 55°C.

The first liquid was introduced into the mixing channel 20 at a flow rate of 0.5 mL/min through the tube 7b via the first fluid inlet channel 10a using the fabricated microfluidic device 1. In addition, the second liquid was introduced into the mixing channel 20 at a flow rate of 2.5 mL/min through the tube 7a via the second fluid inlet channel 10b.

In addition, the second liquid was introduced into the mixing channel 20 at a flow rate of 2.5 mL/min through the tube 7c via the third fluid inlet channel 10c. Accordingly, the first liquid and the second liquid were mixed in the mixing channel 20. Then, the fluid discharged from the mixing channel 20 through the tube 7e via the main fluid outlet channel 31c was collected in a container. The Reynolds number in the mixing channel 20 was set to 228.

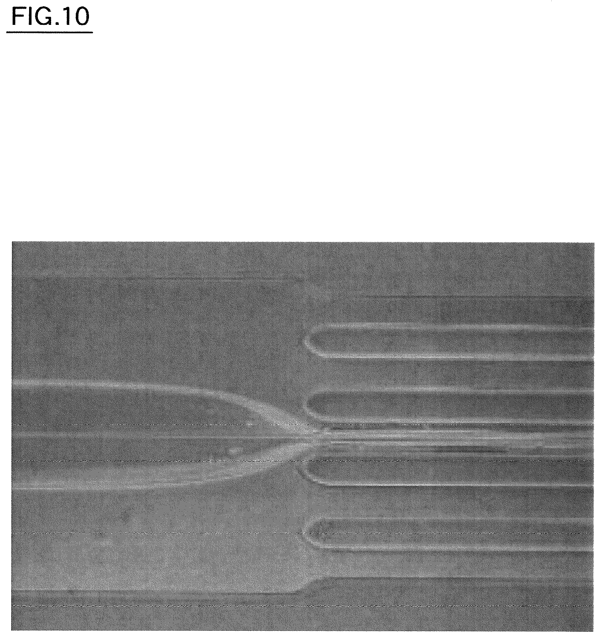

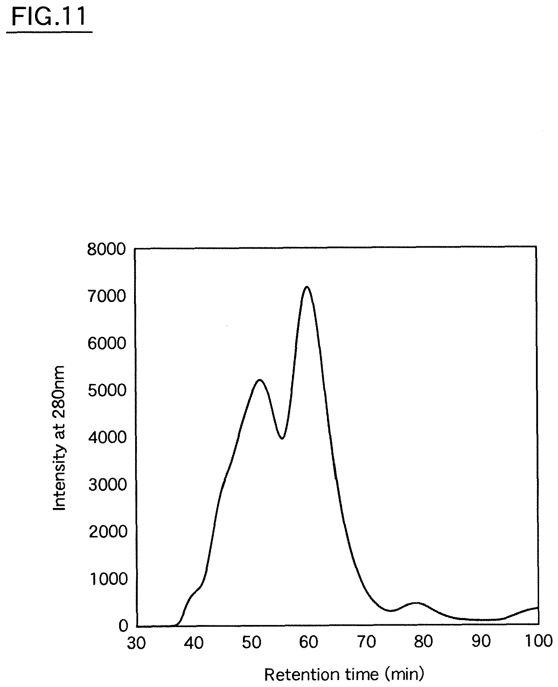

The state of flow of the fluid flowing through the mixing channel 20 was observed under the above conditions. The result is shown in Fig. 10. In addition, in the same method as that in Example 15, the collected solution was dialyzed against PBS for two hours, and the obtained sample was centrifuged at 20000 x g for 15 minutes at 24°C to obtain a supernatant. Then, the supernatant was analyzed by size exclusion chromatography using a column (GE Healthcare: HiLoad 16/60 Superdex 200 prepgrade). The result is shown in Fig. 11.

As shown in Fig. 10, it was confirmed that the fluid (the first liquid and the second liquid) flowing through the mixing channel 20 was discharged from the mixing channel 20 through the main fluid outlet channel 31c (see Fig. 2). It should be noted that, when the main fluid outlet channels 31a, 31b, 31d, and 31e are blocked, the flow velocity of the fluid in the main fluid outlet channel 31c becomes approximately 10 times higher, and the Reynolds number in the main fluid outlet channel 31c becomes approximately 911.

Although detailed figures are omitted, even when the main fluid outlet channels 31a, 31b, 31d, and 31e were blocked, the flow of the first liquid immediately after entering the mixing channel 20 through the first fluid inlet channel 10a became asymmetric such that the flow of the first liquid was biased toward one of the two side walls of the mixing channel 20. Meanwhile, the boundary between the microvortex region 50 and the regions on both sides thereof (the regions mainly containing the second liquid) disappeared.

In addition, as shown in Fig. 11, a large peak was confirmed around a retention time of 50 minutes, and it was confirmed that the particles having a smaller particle size represented by the peak around a retention time of 50 minutes were generated. In addition, a large peak was confirmed around a retention time of 60 minutes, and it was confirmed that the particles having a smaller particle size represented by the peak around a retention time of 60 minutes were generated.

In the column, the peak around a retention time of 50 minutes is attributed to DSPC-rHDL having a particle size of around 15 nm, and the peak around a retention time of 60 minutes is attributed to DSPC-rHDL having a particle size of around 10 nm. In this way, it was confirmed that DSPC-rHDL having a particle size of around 10 nm was generated by restricting the fluid outlet channel 30 (that is, by blocking the main fluid outlet channels 31a, 31b, 31d, and 31e such that the fluid flowed only through the single main fluid outlet channel 31c). This is considered to be due to the increase in the Reynolds number in the fluid outlet channel 30 (the main fluid outlet channel 31c).

Example 18

Instead of the microfluidic device 1 shown in Figs. 1 and 2, the microfluidic device 1a shown in Figs. 12 and 13 was fabricated, and the state of flow of the fluid flowing through the mixing channel 20 was observed in the same manner as in Example 1, except that the following conditions were employed.

In this example, similar to Example 1, the depth of the groove 9a was set to 0.2 mm, the lateral width of each of the main fluid inlet channels 11a to 11c was set to 0.1 mm, and the length of each of the main fluid inlet channels 11b and 11c was set to approximately 5 mm; however, the length of the main fluid inlet channel 11a was set to be longer than that of each of the main fluid inlet channels 11b and 11c. The lateral width of each of the expansion channels 14a to 14c was set to 1 mm. In addition, the spacing (the center-to-center distance) between the main fluid inlet channels 11a to 11c was set to 0.45 mm. The lateral width and the length of the mixing channel 20 were set to 1 mm and 24 mm, respectively. The fluid outlet channel 30 was not provided.

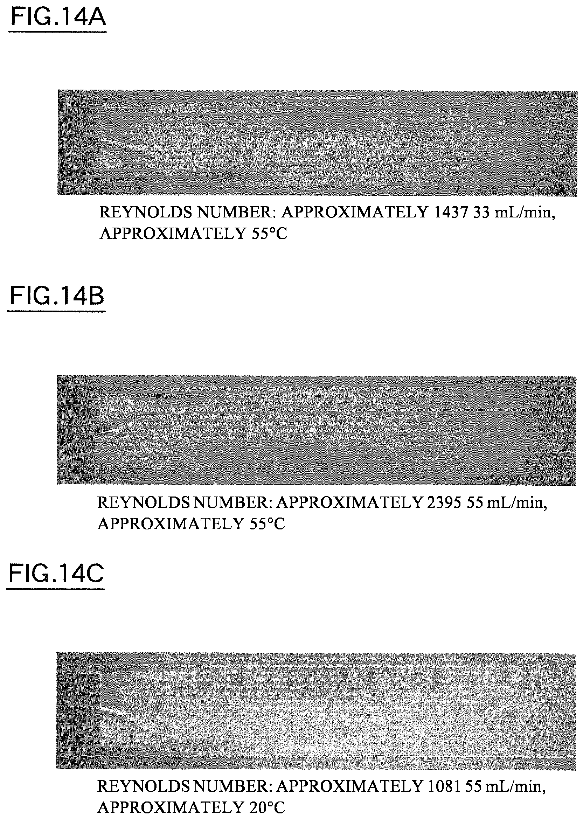

In introducing the first liquid and the second liquid into the microfluidic device 1a, the flow rate ratio of the second liquid (the sum of the second liquid flowing through the second fluid inlet channel 10b and the second liquid flowing through the third fluid inlet channel 10c) to the first liquid was set to 10:1, the flow rate ratio of the second liquid flowing through the second fluid inlet channel 10b to the second liquid flowing through the third fluid inlet channel 10c was set to 10:10, the temperature of the mixing channel 20 was set to approximately 55°C, the total flow rate of the first liquid and the second liquid (the flow rate of the fluid flowing through the mixing channel 20) was set to 33 mL/min, and the Reynolds number in the mixing channel 20 was set to 1437. The first liquid consisting of ethanol was delivered using a syringe pump (Chemyx Corporation: Fusion 200), and the second liquid consisting of pure water was delivered using a syringe pump (Chemyx Corporation: Fusion 6000). The result is shown in Fig. 14(a).

The flow shown in Fig. 14(a) was close to the flow shown in Fig. 5(h), and it could be confirmed that the flow of the first liquid immediately after entering the mixing channel 20 through the first fluid inlet channel 10a became asymmetric such that the flow of the first liquid was more sharply biased toward one of the two side walls of the mixing channel 20. This result showed that, similar to Example 8 and the like, the mixing of the first liquid and the second liquid proceeded at an earlier stage (further upstream in the mixing channel 20).

That is, it could be confirmed that the boundary 70 as shown in Fig. 4(b) disappeared at the initial stage where the first liquid and the second liquid flowed into the mixing channel 20, and the uniform mixing region was more easily formed at an early stage on the upstream side. Therefore, it was showed that the first liquid and the second liquid were more easily mixed; therefore, the mixing efficiency of the first liquid and the second liquid was improved.

Example 19

The total flow rate of the first liquid and the second liquid was set to 55 mL/min, and the Reynolds number in the mixing channel 20 was set to 2395. The other experimental conditions were set to be the same as those in Example 18. The result is shown in Fig. 14(b). As shown in Fig. 14(b), the flow of the first liquid immediately after entering the mixing channel 20 through the first fluid inlet channel 10a became asymmetric such that the flow of the first liquid was more sharply biased toward one of the two side walls of the mixing channel 20 compared to that shown in Fig. 14(a).

In addition, it could be confirmed that the boundary 70 as shown in Fig. 4(b) disappeared at a further initial stage where the first liquid and the second liquid flowed into the mixing channel 20, and the uniform mixing region was more easily formed at an early stage on the upstream side. Therefore, it was showed that the first liquid and the second liquid were more easily mixed; therefore, the mixing efficiency of the first liquid and the second liquid was improved.

Example 20

The total flow rate of the first liquid and the second liquid was set to 55 mL/min, the temperature of the mixing channel 20 was set to approximately 20°C, and the Reynolds number in the mixing channel 20 was set to 1081. The other experimental conditions were set to be the same as those in Example 18. The result is shown in Fig. 14(c). As shown in Fig. 14(c), the flow of the first liquid immediately after entering the mixing channel 20 through the first fluid inlet channel 10a became asymmetric such that the flow of the first liquid was sharply biased toward one of the two side walls of the mixing channel 20 in substantially the same manner as that shown in Fig. 14(a).

In addition, it could be confirmed that, even around a room temperature of 20°C, by setting the Reynolds number to, for example, 1000 or greater, the boundary 70 as shown in Fig. 4(b) disappeared at a further initial stage where the first liquid and the second liquid flowed into the mixing channel 20, and the uniform mixing region was more easily formed at an early stage on the upstream side. Therefore, it was showed that the first liquid and the second liquid were more easily mixed; therefore, the mixing efficiency of the first liquid and the second liquid was improved.

Example 21

Instead of the base chip 2a shown in Fig. 13, a microfluidic device was fabricated using the base chip 2a1 shown in Fig. 15, and the state of flow of the fluid flowing through the mixing channel 20 was observed in the same manner as in Example 18, except that the following conditions were employed.

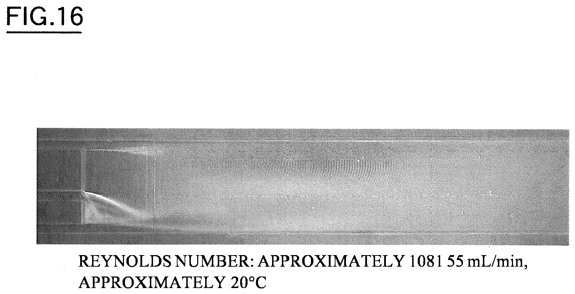

In this example, the centerline (long along the X-axis) of the main fluid inlet channel 11a was offset by approximately 0.1 mm in the Y-axis direction with respect to the centerline of the mixing channel 20 such that the centerline of the main fluid inlet channel 11a approached the main fluid inlet channel 11c. The temperature of the mixing channel 20 was set to approximately 20°C, the total flow rate of the first liquid and the second liquid (the flow rate of the fluid flowing through the mixing channel 20) was set to 55 mL/min, and the Reynolds number in the mixing channel 20 was set to 1081. The result is shown in Fig. 16.

The flow shown in Fig. 16 was close to the flow shown in Fig. 14(c), and it could be confirmed that the flow of the first liquid immediately after entering the mixing channel 20 through the first fluid inlet channel 10a became asymmetric such that the flow of the first liquid was more sharply biased toward one of the two side walls of the mixing channel 20. This result showed that, similar to Fig. 14(c) and the like, the mixing of the first liquid and the second liquid proceeded at an earlier stage (further upstream in the mixing channel 20).

That is, it could be confirmed that the boundary 70 as shown in Fig. 4(b) disappeared at the initial stage where the first liquid and the second liquid flowed into the mixing channel 20, and the uniform mixing region was more easily formed at an early stage on the upstream side. Therefore, it was showed that the first liquid and the second liquid were more easily mixed; therefore, the mixing efficiency of the first liquid and the second liquid was improved.

Example 22

Instead of the base chip 2a1 shown in Fig. 15, a microfluidic device was fabricated using the base chip 2a2 shown in Fig. 17, and the state of flow of the fluid flowing through the mixing channel 20 was observed in the same manner as in Example 21, except that the following conditions were employed.

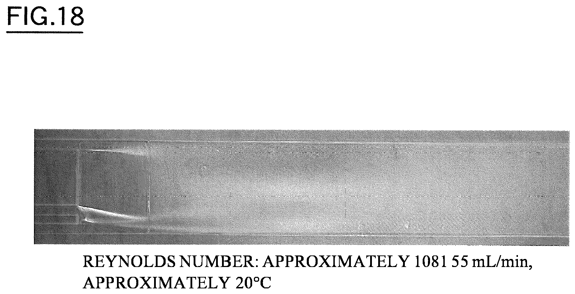

In this example, the centerline (long along the X-axis) of the main fluid inlet channel 11a was offset by approximately 0.25 mm in the Y-axis direction with respect to the centerline of the mixing channel 20 such that the centerline of the main fluid inlet channel 11a approached the main fluid inlet channel 11c. The result is shown in Fig. 18.

The flow shown in Fig. 18 was close to the flow shown in Fig. 16, and it could be confirmed that the flow of the first liquid immediately after entering the mixing channel 20 through the first fluid inlet channel 10a became asymmetric such that the flow of the first liquid was more sharply biased toward one of the two side walls of the mixing channel 20. This result showed that, similar to Fig. 16 and the like, the mixing of the first liquid and the second liquid proceeded at an earlier stage (further upstream in the mixing channel 20).

That is, it could be confirmed that the boundary 70 as shown in Fig. 4(b) disappeared at the initial stage where the first liquid and the second liquid flowed into the mixing channel 20, and the uniform mixing region was more easily formed at an early stage on the upstream side. Therefore, it was showed that the first liquid and the second liquid were more easily mixed; therefore, the mixing efficiency of the first liquid and the second liquid was improved.

Example 23

Instead of the microfluidic device 1 shown in Figs. 1 and 2, the microfluidic device 1a shown in Figs. 12 and 13 was fabricated, and reconstituted high-density lipoprotein (rHDL) was produced in the same manner as in Example 15, except that the following conditions were employed.

A 5 mg/mL lipid solution in which DSPC was dissolved in ethanol was used as the first liquid. A 200 μg/mL protein solution in which apolipoprotein A-I was dissolved in phosphate-buffered saline (PBS) containing 2M Urea was used as the second liquid. The density of the second liquid (2M Urea solution) at 25°C was approximately 1.03, and the viscosity was approximately 1.09. In addition, the temperature of the mixing channel 20 was approximately 20°C. The flow rate of the first liquid was 5 mL/min, the flow rate of the second liquid was 50 mL/min, and the total flow rate of the first liquid and the second liquid was 55 mL/min. The Reynolds number in the mixing channel 20 was approximately 1028.

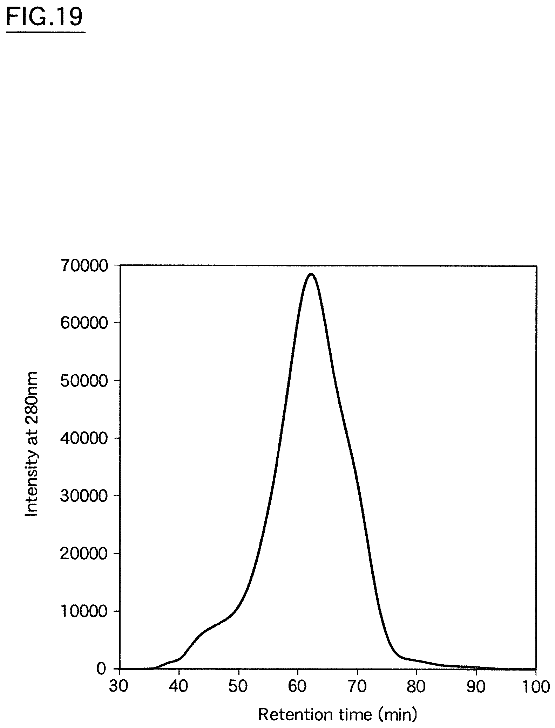

The collected liquid was placed in a centrifugal filter unit (Merck Corporation: Amicon Ultra-15, 50kDa) and was centrifuged at 5000 G for 20 minutes at 4°C to concentrate the collected liquid approximately 22-fold, and then the collected liquid was diluted approximately 27-fold with PBS to lower the concentration of ethanol to approximately 0.3%, and then the analysis was performed. The result of size exclusion chromatography are shown in Fig. 19.

The column used is obtained by packing an empty column (Cytiva Corporation: XK 16/70 column) with a carrier (Cytiva Corporation: Superdex 200 prepgrade), and the retention time is shown as the value divided by 1.06 to correct for a shift in retention time with respect to the above-described HiLoad 16/60 Superdex 200 prepgrade. The peak around a retention time of 60 minutes was dominant, and it was confirmed that the particles having a smaller particle size were generated with high efficiency. The average hydrodynamic diameter was measured as 9.6 nm using a particle size distribution analyzer. The yield on a protein basis was 96%.

From the results of Example 15, it is confirmed that the particles having a smaller particle size represented by the peak around a retention time of 60 minutes are rHDL using DSPC as a lipid (DSPC-rHDL). For this reason, it is considered that the particles having a smaller particle size represented by the peak around a retention time of 60 minutes shown in Fig. 19 obtained in this embodiment are rHDL using DSPC as a lipid (DSPC-rHDL).

In this way, it was confirmed that, in the method for manufacturing nanoparticles according to the present example, the reconstitution of DSPC-rHDL, which was difficult to manufacture using conventional methods for manufacturing nanoparticles using microfluidic devices, was enabled even around room temperature.

Example 24

Lipid nanoparticles other than DSPC-rHDL and lipid nanoparticles consisting of DSPC and cholesterol were produced in the same manner as in Example 23, except that the following conditions were employed.

As the first liquid, ethanol containing 10 mg/mL of DSPC and 4.89 mg/mL of cholesterol was allowed to flow at 5 mL/min, as the second liquid, PBS was allowed to flow at 50 mL/min, the total flow rate of the first liquid and the second liquid was 55 mL/min, and the temperature of the mixing channel was approximately 20°C.

After dialysis, the average hydrodynamic diameter was measured as 30.1 nm. It was showed that lipid nanoparticles consisting of DSPC and cholesterol could be produced.

EXPLANATIONS OF LETTERS OR NUMERALS

1, 1a MICROFLUIDIC DEVICE
2a, 2al, 2a2 BASE CHIP
2b COVER CHIP
3a to 3g THROUGH-HOLE
4a, 4b CLAMPING PLATE
5a to 5g THROUGH-HOLE
6 SCREW HOLE
7a to 7d, 7d1, 7d2, 7e, 7g TUBE
70a to 70g CONNECTOR
8a SCREW
8b NUT
9, 9a GROOVE
10a to 10C FIRST FLUID INLET CHANNEL TO THIRD FLUID INLET CHANNEL
11a to 11c MAIN FLUID INLET CHANNEL
12a to 12c DOWNSTREAM EXPANSION CHANNEL
13a to 13c UPSTREAM EXPANSION CHANNEL
14a to 14c EXPANSION CHANNEL
20 MIXING CHANNEL
30 FLUID OUTLET CHANNEL
31a to 31e MAIN FLUID OUTLET CHANNEL
32a to 32c OUTLET EXPANSION CHANNEL
40a to 40g TERMINAL PORTION
50 MICROVORTEX REGION
60 FIRST BOUNDARY
70 SECOND BOUNDARY

**Claims**

1. A method for manufacturing nanoparticles, comprising:

   introducing a first liquid containing at least one material into a mixing channel through a first fluid inlet channel of a microfluidic device, and introducing a second liquid into the mixing channel through a second fluid inlet channel and a third fluid inlet channel on both sides of the first fluid inlet channel,
   wherein a Reynolds number in the mixing channel is set to a predetermined value or greater, and a flow of the first liquid in the mixing channel becomes asymmetric such that the flow of the first liquid immediately after entering the mixing channel through the first fluid inlet channel is biased toward one of two side walls of the mixing channel.

2. The method for manufacturing nanoparticles according to claim 1,
   wherein the Reynolds number is 151 or greater.

3. The method for manufacturing nanoparticles according to claim 2,
   wherein the Reynolds number is 281 or greater.

4. The method for manufacturing nanoparticles according to any one of claims 1 to 3,

   wherein a spacing between the first fluid inlet channel and the second fluid inlet channel is greater than a lateral width of each of the first fluid inlet channel, the second fluid inlet channel, and the third fluid inlet channel, and a spacing between the first fluid inlet channel and the third fluid inlet channel is greater than the lateral width of each of the first fluid inlet channel, the second fluid inlet channel, and the third fluid inlet channel.

5. The method for manufacturing nanoparticles according to any one of claims 1 to 3, further comprising:
   discharging the first liquid and the second liquid from the mixing channel through a fluid outlet channel located downstream of the mixing channel.

6. The method for manufacturing nanoparticles according to claim 5,
   wherein the fluid outlet channel is composed of a single fluid outlet channel having a lateral width narrower than a lateral width of the mixing channel.

7. The method for manufacturing nanoparticles according to any one of claims 1 to 3,
   wherein a temperature of the mixing channel is 45°C or higher.

8. The method for manufacturing nanoparticles according to any one of claims 1 to 3,
   wherein a temperature of the mixing channel is room temperature or higher.

9. The method for manufacturing nanoparticles according to any one of claims 1 to 3,
   wherein the at least one material contains a lipid.

10. The method for manufacturing nanoparticles according to any one of claims 1 to 3,
    wherein the second liquid is an aqueous solvent, and contains at least one water-soluble material.

11. The method for manufacturing nanoparticles according to claim 10,
    wherein the at least one water-soluble material contains at least one of a protein and a peptide.

FIG.1

FIG.2

FIG.3

EP 4 775 530 A1

## FIG.4A

## FIG.4B

## FIG.4C

## FIG.5A

REYNOLDS NUMBER: APPROXIMATELY 151 7.7 mL/min,
APPROXIMATELY 20°C

## FIG.5B

REYNOLDS NUMBER: APPROXIMATELY 173 8.8 mL/min,
APPROXIMATELY 20°C

## FIG.5C

REYNOLDS NUMBER: APPROXIMATELY 281 14.3 mL/min,
APPROXIMATELY 20°C

## FIG.5D

REYNOLDS NUMBER: APPROXIMATELY 306 16.5 mL/min,
APPROXIMATELY 20°C

## FIG.5E

REYNOLDS NUMBER: APPROXIMATELY 345 18.5 mL/min,
APPROXIMATELY 20°C

## FIG.5F

REYNOLDS NUMBER: APPROXIMATELY 383 8.8 mL/min,
APPROXIMATELY 55°C

## FIG.5G

REYNOLDS NUMBER: APPROXIMATELY 479 11 mL/min,
APPROXIMATELY 55°C

# FIG.5H

REYNOLDS NUMBER: APPROXIMATELY 575 13.2 mL/min,
APPROXIMATELY 55°C

# FIG.5I

REYNOLDS NUMBER: APPROXIMATELY 108 5.5 mL/min,
APPROXIMATELY 20°C

# FIG.5J

REYNOLDS NUMBER: APPROXIMATELY 130 6.6 mL/min,
APPROXIMATELY 20°C

## FIG.6A

REYNOLDS NUMBER: APPROXIMATELY 175 9.4 mL/min,
APPROXIMATELY 20°C

## FIG.6B

REYNOLDS NUMBER: APPROXIMATELY 197 4.7 mL/min,
APPROXIMATELY 55°C

## FIG.6C

REYNOLDS NUMBER: APPROXIMATELY 316 7.5 mL/min,
APPROXIMATELY 55°C

# FIG.6D

REYNOLDS NUMBER: APPROXIMATELY 395 9.4 mL/min,
APPROXIMATELY 55°C

# FIG.6E

REYNOLDS NUMBER: APPROXIMATELY 474 11.3 mL/min,
APPROXIMATELY 55°C

## FIG.7

# FIG.8

FIG.9

FIG.10

FIG.11

FIG.12

FIG.13

EP 4 775 530 A1

## FIG.14A

REYNOLDS NUMBER: APPROXIMATELY 1437 33 mL/min,
APPROXIMATELY 55°C

## FIG.14B

REYNOLDS NUMBER: APPROXIMATELY 2395 55 mL/min,
APPROXIMATELY 55°C

## FIG.14C

REYNOLDS NUMBER: APPROXIMATELY 1081 55 mL/min,
APPROXIMATELY 20°C

FIG.15

EP 4 775 530 A1

FIG.16

REYNOLDS NUMBER: APPROXIMATELY 1081 55 mL/min,
APPROXIMATELY 20°C

EP 4 775 530 A1

# FIG.17

FIG.18

REYNOLDS NUMBER: APPROXIMATELY 1081 55 mL/min,
APPROXIMATELY 20°C

# FIG.19

## INTERNATIONAL SEARCH REPORT

| International application No. |
| --- |
| **PCT/JP2024/031996** |

**A. CLASSIFICATION OF SUBJECT MATTER**

*B82B 3/00*(2006.01)i; *B01F 33/3011*(2022.01)i; *B01J 13/02*(2006.01)i; *B01J 19/00*(2006.01)i; *C12N 15/88*(2006.01)i
FI:  B82B3/00; B01J13/02; B01F33/3011; C12N15/88 Z; B01J19/00 321

According to International Patent Classification (IPC) or to both national classification and IPC

**B. FIELDS SEARCHED**

Minimum documentation searched (classification system followed by classification symbols)

A61J3/07; A61K9/10-133,48-66; A61K38/00-58; B01F33/30-3039; B01J13/00-22; B01J19/00,24; B81B1/00; B82B3/00; C07B61/00; C12N15/88

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2005/023704 A1 (NATIONAL INSTITUTE OF ADVANCED INDUSTRIAL SCIENCE AND TECHNOLOGY) 17 March 2005 (2005-03-17) | 1-11 |
| A | JP 2009-291783 A (COMMISSARIAT A L'ENERGIE ATOMIQUE) 17 December 2009 (2009-12-17) | 1-11 |
| A | JP 2004-122107 A (TOSOH CORP.) 22 April 2004 (2004-04-22) | 1-11 |
| A | JP 2003-215741 A (FUJI PHOTO FILM CO., LTD.) 30 July 2003 (2003-07-30) | 1-11 |
| A | WO 2018/123883 A1 (KAO CORPORATION) 05 July 2018 (2018-07-05) | 1-11 |
| A | JP 2013-255912 A (CHIBA UNIV) 26 December 2013 (2013-12-26) | 1-11 |
| A | US 8563325 B1 (BARTSCH, Michael) 22 October 2013 (2013-10-22) | 1-11 |

☐ Further documents are listed in the continuation of Box C.      ☑ See patent family annex.

\*    Special categories of cited documents:
"A"    document defining the general state of the art which is not considered to be of particular relevance
"D"    document cited by the applicant in the international application
"E"    earlier application or patent but published on or after the international filing date
"L"    document which may throw doubts on priority claim(s) or which is cited to establish the publication date of another citation or other special reason (as specified)
"O"    document referring to an oral disclosure, use, exhibition or other means
"P"    document published prior to the international filing date but later than the priority date claimed

"T"    later document published after the international filing date or priority date and not in conflict with the application but cited to understand the principle or theory underlying the invention
"X"    document of particular relevance; the claimed invention cannot be considered novel or cannot be considered to involve an inventive step when the document is taken alone
"Y"    document of particular relevance; the claimed invention cannot be considered to involve an inventive step when the document is combined with one or more other such documents, such combination being obvious to a person skilled in the art
"&"    document member of the same patent family

| Date of the actual completion of the international search | Date of mailing of the international search report |
| --- | --- |
| **18 November 2024** | **03 December 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/031996**

| Patent document cited in search report | | | Publication date (day/month/year) | Patent family member(s) | | | Publication date (day/month/year) |
|---|---|---|---|---|---|---|---|
| WO | 2005/023704 | A1 | 17 March 2005 | US | 2007/0128350 | A1 | |
| JP | 2009-291783 | A | 17 December 2009 | US | 2009/0283456 | A1 | |
| | | | | EP | 2127748 | A1 | |
| | | | | FR | 2931085 | A1 | |
| JP | 2004-122107 | A | 22 April 2004 | US | 2003/0201022 | A1 | |
| | | | | EP | 1358931 | A2 | |
| JP | 2003-215741 | A | 30 July 2003 | (Family: none) | | | |
| WO | 2018/123883 | A1 | 05 July 2018 | US | 2020/0078272 | A1 | |
| | | | | EP | 3536399 | A1 | |
| | | | | TW | 201828914 | A | |
| JP | 2013-255912 | A | 26 December 2013 | (Family: none) | | | |
| US | 8563325 | B1 | 22 October 2013 | (Family: none) | | | |

Form PCT/ISA/210 (patent family annex) (July 2022)

**REFERENCES CITED IN THE DESCRIPTION**

**Patent documents cited in the description**

- US 10864162 B